# EUROPÄISCHE PATENTSCHRIFT

(11) **EP 2 037 972 B1**
(45) Veröffentlichungstag und Bekanntmachung des Hinweises auf die Patenterteilung: **21.09.2011**
(21) Anmeldenummer: 07785971.8
(22) Anmeldetag: 10.07.2007
(51) Int. Cl.: A61L 24/10

(54) **VERWENDUNG VON GELATINE UND TRANSGLUTAMINASE ZUR HERSTELLUNG EINES VERNETZENDEN MEDIZINISCHEN KLEBERS**
USE OF GELATIN AND TRANSGLUTAMINASE FOR PRODUCING CROSS-LINKING MEDICAL GLUES
UTILISATION DE GÉLATINE ET DE TRANSGLUTAMINASE POUR PRODUIRE UN ADHÉSIF MÉDICAL À RÉTICULATION

(30) Priorität: 10.07.2006 DE 102006033167
(43) Veröffentlichungstag der Anmeldung: 25.03.2009
(73) Patentinhaber: GELITA AG, 69412 Eberbach (DE); Tetec-Tissue Engineering Technologies Aktiengesellschaft, 72770 Reutlingen (DE)
(72) Erfinder: GAISSMAIER, Christoph, 72127 Kusterdingen (DE); AHLERS, Michael, 69412 Eberbach (DE)
(74) Vertreter: Wössner, Gottfried
(86) Internationale Anmeldenummer: PCT/EP2007/006105
(87) Internationale Veröffentlichungsnummer: WO 2008/006545

(56) Entgegenhaltungen:
- WO-A-97/29715
- WO-A-2005/111121
- WO-A-2006/083384
- WO-A-2008/006544
- US-A- 6 007 613
- US-A1- 2002 015 724
- MCDERMOTT, M; CHEN, T; WILLIAMS, C; MARKLEY, K; PAYNE, G: "Mechanical properties of biomimetic tissue adhesive based on the microbial transglutaminase-catalyzed crosslinking of gelatin" BIOMACROMOLECULES, Bd. 5, 21. April 2004 (2004-04-21), Seiten 1270-1279, XP002494450

## Beschreibung

Die vorliegende Erfindung betrifft eine neuartige Verwendung von Gelatine und einem Vernetzungsmittel zur Herstellung eines vernetzenden medizinischen Klebers, der ein vernetztes Gelatinegel in einem Applikationsbereich des menschlichen oder tierischen Körpers bildet.

In verschiedenen Bereichen der Medizin besteht der Bedarf nach Zusammensetzungen mit klebenden Eigenschaften, d.h. sogenannten medizinischen Klebern. Anwendungsbereiche derartiger Kleber sind zum Beispiel die Fixierung von Geweben, Gewebeteilen oder Organen, mitunter als Alternative und/oder Ergänzung zum chirurgischen Nähen, sowie das Abdeckung bzw. Verschließen von Wundflächen oder Stillen von Blutungen, sowohl innerlich als auch äußerlich.

Als Grundlage für solche Zusammensetzungen, die im oder am Körper eingesetzt werden, bieten sich in erster Linie biologisch abbaubare Materialien an, die nach einer bestimmten Zeit, wenn die Kleberfunktion nicht mehr benötigt wird, abgebaut und vom Körper resorbiert werden.

In der Praxis sind medizinische Kleber bekannt, die verschiedene Komponenten humanen Ursprungs umfassen und auf dem Prinzip der natürlichen Blutgerinnung beruhen. Die Hauptkomponenten solcher Systeme sind in der Regel Thrombin, Fibrinogen und Blutgerinnungsfaktor XIII, wobei unmittelbar nach dem Mischen dieser Komponenten, d.h. innerhalb weniger Sekunden, ein festes Gel entsteht. Dieses Gel weist jedoch in verschiedener Hinsicht, insbesondere bezüglich der Kinetik der Gelbildung und der Klebrigkeit, nicht die für die meisten Anwendungen gewünschten Eigenschaften auf.

Bei der Verwendung von Komponenten humanen Ursprungs besteht zudem ein gewisses Risiko im Hinblick auf die Übertagung von Infektionskrankheiten. Dieses Problem stellt sich somit auch bei der in der WO 94/01508 A1 beschriebenen Kleberzusammensetzung, bei der ein Plasmaprotein oder ein globuläres Protein mit einem di- oder polyfunktionalen Aldehyd vernetzt wird.

Es sind des weiteren auch Klebersysteme bekannt, die auf einer Vernetzung von Rinderserumalbumin mit Glutaraldehyd basieren. Der Einsatz von Glutaraldehyd als Vernetzungsmittel ist allerdings aufgrund zytotoxischer Effekte zum Teil problematisch.

Die DE 101 52 407 A1 offenbart eine Zusammensetzung aus mindestens zwei untereinander chemisch vernetzbaren Komponenten zum Verkleben von biologischem Gewebe, umfassend eine Lösung eines aminogruppentragenden Polymers und eine Lösung eines Aldehyds mit mindestens drei Aldehydgruppen. Als mögliche aminogruppentragende Komponenten werden dabei sowohl Polymere natürlichen Ursprungs, wie z.B. deacetyliertes Chitin, als auch synthetische Polymere genannt.

In der WO 97/29715 A1 werden Zusammensetzung zum Versiegeln von Gewebe beschrieben, die Kollagen oder Gelatine, ein oder mehrere Vernetzungsmittel und gegebenenfalls einen Weichmacher umfassen. Als Vernetzungsmittel werden dabei Aldehyde, insbesondere Di- oder Polyaldehyde, eingesetzt.

Die US 2002/0015724 A1 beschreibt medizinische Zusammensetzungen zum Versiegeln von Gefäßen und als Wundauflage, wobei die Zusammensetzungen auf der Basis von polymerisiertem Kollagen vom Typ I und/oder vom Typ III gebildet sind. Dabei werden Kollagenmonomere rekombinant hergestellt und mit einem geeigneten Agens polymerisiert.

M. McDermott et al. (Biomacromolecules 2004, 5, 1270-1279) beschreiben die mechanischen Eigenschaften eines Gewebeklebers auf der Basis von Gelatine, die mit mikrobieller Transglutaminase vernetzt wird. Für den Kleber wird Gelatine vom Typ A mit einem Bloomwert von 175 bzw. 300 g eingesetzt.

Die US 6,007,613 offenbart einen biologischen Klebstoff mit mindestens zwei Komponenten, wobei die erste, halbflüssige Komponente eine wässrige Gelatinelösung umfasst und die zweite Komponente, die in Form eines Gels vorliegen kann, einen Aldehyd umfasst.

Die WO 2006/083384 A1 beschreibt einen Gewebekleber, der hergestellt ist durch Vernetzen von Albumin und/oder Gelatine mit bestimmten Polyaminen und/oder Polycarboxylaten unter Verwendung eines wasserlöslichen Carbodiimids.

Die Aufgabe der vorliegenden Erfindung besteht darin, einen medizinischen Kleber mit verbesserten Eigenschaften vorzuschlagen.

Zur Lösung dieser Aufgabe wird erfindungsgemäß die Verwendung von Gelatine und Transglutaminase als Vernetzungsmittel zur Herstellung eines vernetzenden medizinischen Klebers der eingangs genannten Art vorgeschlagen, der im Applikationsbereich ein vernetztes Gelatinegel bildet, wobei
(i) die Gelatine und die Transglutaminase zur Bildung des vernetzenden medizinischen Klebers miteinander gemischt werden, welcher anschließend in den Applikationsbereich verabreicht wird; oder
(ii) die Gelatine und die Transglutaminase in voneinander getrennter Form bereitgestellt und gleichzeitig oder aufeinander folgend unter Bildung des vernetzenden medizinischen Klebers in den Applikationsbereich verabreicht werden,
und wobei die Gelatine eine Virkosität von 7 mPa·s oder mehr aufweist, gemessen in einer 6,7 Gew.%igen Lösung bei 60°C.

Der medizinische Kleber gemäß der vorliegenden Erfindung umfasst als Hauptkomponente Gelatine als ein körperverträgliches, biologisch abbaubares Material. Der Erfindung liegt das Prinzip zu Grunde, die Gelatine in im Wesentlichen unvernetzter, gelöster Form zu verabreichen, und erst im Applikationsbereich durch die Einwirkung eines Vernetzungsmittels in ein vernetztes Gelatinegel, welches als medizinischer Kleber wirkt, zu überführen.

Ein besonderer Vorteil des erfindungsgemäßen medizinischen Klebers liegt darin, dass dieser kontrolliert vernetzbar ist. Kontrollierte Vernetzbarkeit ist im Rahmen der Erfindung dahingehend zu verstehen, dass die maximale Festigkeit des vernetzten Gelatinegels nicht unmittelbar (d.h. innerhalb weniger Sekunden) nach dem Mischen der Gelatine und des Vernetzungsmittels erreicht wird, sondern dass die Gelbildung verzögert einsetzt und die Gelfestigkeit innerhalb eines bestimmten Zeitraums kontinuierlich ansteigt. Die Kinetik und das Ausmaß der Gelbildung können dabei mittels verschiedener Parameter beeinflusst bzw. kontrolliert werden, welche weiter unten im Einzelnen beschrieben sind.

Der erfindungsgemäße medizinische Kleber benötigt außer der Gelatine und dem Vernetzungsmittel keine weiteren Komponenten, die zur Bildung des vernetzten Gelatinegels beitragen. Er kann damit wesentlich einfacher aufgebaut werden als das oben beschriebene System auf der Basis von Thrombin und Fibrinogen, welches notwendigerweise eine Vielzahl weiterer Komponenten umfasst. Wenngleich die Funktion des vorliegenden medizinischen Klebers mittels nur zweier Komponenten, d.h. Gelatine und einem Vernetzungsmittel, verwirklicht werden kann, schließt dies die Anwesenheit weiterer Bestandteile nicht aus. Durch solche zusätzliche Komponenten können z.T. weitere vorteilhafte Effekte erzielt werden, wie dies weiter unten näher beschrieben wird.

Ein weiterer Vorteil der Erfindung besteht darin, dass der medizinische Kleber keine Komponenten humanen Ursprungs umfassen muss, wodurch die Herstellung einfacher und kostengünstiger wird.

Die oben beschriebene kontrollierte Vernetzung bietet den Vorteil, dass dem behandelnden Arzt nach dem Mischen der Gelatine und des Vernetzungsmittels ausreichend Zeit bleibt, um den medizinischen Kleber auf den Applikationsbereich aufzutragen, in diesen zu injizieren oder in sonstiger Weise zu verabreichen. Dies ist insbesondere bei schlecht zugänglichen Applikationsbereichen, z.B. im Rahmen von Operationen, von Bedeutung, da sich hier die Verabreichung oft schwierig gestaltet und eine gewisse Zeit in Anspruch nimmt. Auch nach dem Beginn der Vernetzungsreaktion ist der medizinische Kleber während einem gewissen Zeitraum noch plastisch verformbar und kann an die Struktur des Applikationsbereichs angepasst werden, beispielsweise an die Oberfläche eines Gewebes oder an eine auszufüllende Kavität. Die Viskosität des Klebers kann während dieses Zeitraums bereits ansteigen. Auch dies ist ein deutlicher Vorteil gegenüber Klebern auf Basis von Thrombin, welche nach dem Mischen der Komponenten praktisch schlagartig aushärten und dann nicht mehr verformt werden können.

Der medizinische Kleber gemäß der vorliegenden Erfindung weist eine hohe Klebrigkeit auf, und zwar nicht nur gegenüber strukturierten Oberflächen wie z.B. Geweben, sondern selbst gegenüber glatten Kunststoffoberflächen (z.B. poliertes Polymethylmethacrylat). Demgegenüber zeigen Zusammensetzungen auf der Basis von Thrombin und Fibrinogen eine wesentlich geringere Klebrigkeit und eine wesentlich verminderte Haftung gegenüber glatten Kunststoffoberflächen.

Ein Grund für die guten und dauerhaften Klebeigenschaften ist darin zu sehen, dass das vernetzte Gelatinegel gemäß der vorliegenden Erfindung im Wesentlichen keine Synerese zeigt, d.h. es kommt nicht zu einer Schrumpfung des Gels unter Austritt von Wasser. Der erfindungsgemäße medizinische Kleber weist somit auch eine hohe Maßhaltigkeit auf, was insbesondere auch bei Ausfüllen von Kavitäten von Vorteil ist.

Der erfindungsgemäße vernetzende Kleber kann in verschiedenen Bereichen der Medizin angewendet werden, so dass je nach Art des Applikationsbereiches unterschiedliche Verabreichungsmethoden bevorzugt sein können.

Bevor die verschiedenen Varianten der Bereitstellung und Verabreichung der Gelatine und des Vernetzungsmittels im Detail beschrieben werden, soll zunächst auf die besonderen Vorteile eingegangen werden, die sich durch die Auswahl von Gelatine als Biomaterial ergeben.

Gelatine ist, im Gegensatz zu Kollagen, in definierter und reproduzierbarer Zusammensetzung sowie in hoher Reinheit erhältlich. Insbesondere enthält sie praktisch keine immunogenen Telopeptide, die Abwehrreaktionen des Körpers auslösen könnten. Insofern weist Gelatine eine hervorragende Gewebe- und Zellverträglichkeit auf, wie sie von anderen resorbierbaren Biomaterialien wie Alginaten oder Chitosan nicht gewährleistet werden kann.

Während unvernetzte Gelatine bei Körpertemperatur (37°C) löslich ist, kann sie, wie bereits erwähnt, durch Vernetzung in eine unter diesen Bedingungen unlösliche gelartige Form, d.h. ein vernetztes Gelatinegel, überführt werden.

Gleichzeitig ist das vernetzte Gelatinegel vollständig resorbierbar, d.h. es wird nach einer bestimmten Zeit im Körper rückstandslos abgebaut. Dabei handelt es sich um einen hydrolytischen Abbau, der gegebenenfalls durch körpereigene Enzyme unterstützt wird.

Grundsätzlich kann im Rahmen der vorliegenden Erfindung Gelatine verschiedener Herkunft eingesetzt werden, wobei porcine Gelatine bevorzugt ist, insbesondere Schweineschwartengelatine. Diese ist in hoher Qualität erhältlich und ist bereits für verschiedene medizinische Anwendungen zugelassen.

Daneben kann auch die Verwendung anderer Gelatinearten wie z.B. Fischgelatine besondere Vorteile bieten. Insbesondere die aus Kaltwasserfischen gewonnene Gelatine zeichnet sich durch eine relativ niedrige Gelierungstemperatur aus, d.h. wässrige Lösungen von (unvernetzter) Fischgelatine bleiben bei niedrigeren Temperaturen flüssig als beispielsweise Lösungen von Schweineschwartengelatine gleicher Konzentration. Diese Tatsache erlaubt es, gelöste Fischgelatine bei Raumtemperatur oder sogar gekühlt bereitzustellen, was die Handhabung gegenüber einer Bereitstellung bei erhöhten Temperaturen von bis zu 37°C vereinfacht.

Um die Bioverträglichkeit des medizinischen Klebers weiter zu verbessern, wird bevorzugt eine Gelatine mit einem besonders geringen Gehalt an Endotoxinen eingesetzt. Bei Endotoxinen handelt sich um Stoffwechselprodukte oder Bruchstücke von Mikroorganismen, welche in dem tierischen Rohmaterial vorkommen.

Der Endotoxingehalt von Gelatine wird in internationalen Einheiten pro Gramm (I.E./g) angegeben und gemäß dem LAL-Test bestimmt, dessen Durchführung in der vierten Ausgabe des Europäischen Arzneibuches (Ph. Eur. 4) beschrieben ist.

Um den Gehalt an Endotoxinen möglichst gering zu halten, ist es vorteilhaft, die Mikroorganismen möglichst frühzeitig im Zuge der Gelatineherstellung abzutöten. Ferner sollten entsprechende Hygienestandards beim Herstellungsprozess eingehalten werden.

Somit kann der Endotoxingehalt von Gelatine durch bestimmte Maßnahmen beim Herstellungsprozess drastisch gesenkt werden. Zu diesen Maßnahmen zählen in erster Linie die Verwendung frischer Rohmaterialien (z.B. Schweineschwarte) unter Vermeidung von Lagerzeiten, die sorgfältige Reinigung der gesamten Produktionsanlage unmittelbar vor Beginn der Gelatineherstellung sowie gegebenenfalls das Auswechseln von Ionenaustauschern und Filtersystemen in der Produktionsanlage.

Die im Rahmen der vorliegenden Erfindung eingesetzte Gelatine weist bevorzugt einen Endotoxingehalt von 1.200 I.E./g oder weniger, noch mehr bevorzugt von 200 I.E./g oder weniger auf. Optimalerweise liegt der Endotoxingehalt bei 50 I.E./g oder weniger, jeweils gemäß dem LAL-Test bestimmt. Im Vergleich hierzu weisen manche handelsübliche Gelatinen Endotoxingehalte von 20.000 I.E./g und mehr auf.

Bei Gelatine, die durch Extraktion von kollagenhaltigen Rohmaterialien gewonnen wird, handelt es sich, wie bereits angesprochen, um ein wasserlösliches Produkt, das insbesondere bei den für die Verabreichung geeigneten Temperaturen, d.h. 37°C oder weniger, in Lösung gebracht werden kann. Diese gelöste Form ist für die Verabreichung besonders vorteilhaft, da die Lösung z.B. auf die Oberfläche eines zu behandelnden Gewebes aufgebracht werden kann und sich dort gleichmäßig verteilt. Um die Gelatine nach der Verabreichung, d.h. im Applikationsbereich des Körpers, in ein Gelatinegel zu überführen, erfolgt erfindungsgemäß eine Vernetzung der Gelatine.

Herrscht im Applikationsbereich eine niedrigere Temperatur, insbesondere eine Temperatur, die unterhalb der Geliertemperatur von Gelatine (ca. 32 bis 33°C) liegt, z.B. 30°C, so beobachtet man überraschenderweise eine beschleunigte Gelierung, obwohl die Vernetzungsaktivität der Transglutaminase bei diesen Temperaturen gegenüber einer Temperatur von 37°C vermindert ist. Dieses Ergebnis wird darauf zurückgeführt, dass hier neben der Vernetzungsreaktion die dem Gelatinematerial eigene Gelbildung zum Tragen kommt und in der Summe eine erhöhte Geliergeschwindigkeit resultiert.

Temperaturen im Applikationsbereich, die weit unter der Geliertemperatur des Gelatinematerials liegen, beispielsweise 25°C, empfehlen sich in der Regel nicht, da es hier zu einer unzureichenden oder ungleichmäßigen Vernetzung des Gelatinematerials kommen kann.

Es sind verschiedene Arten von Vernetzungsmitteln bekannt, die die Gelatine durch inter- und/oder intramolekulare Verknüpfungen in ein Gelatinegel überführen, welches bei Temperaturen von 37°C oder weniger unlöslich ist. Bei diesen Verknüpfungen zwischen den Gelatinemolekülen kann es sich sowohl um kovalente Bindungen handeln als auch um eine Komplexbildung, die beispielsweise auf ionischen Wechselwirkungen, Wasserstoffbrücken oder Vander-Waals-Kräften beruht.

Vor dem Hintergrund der physiologischen Verträglichkeit wird bei der vorliegenden Erfindung auf Transglutaminase als enzymatisches Vernetzungsmittel zurückgegriffen. Dieses Enzym, das in Tieren, Pflanzen und Bakterien vorkommt, katalysiert die Hydrolyse der Amidbindung von Glutaminresten und die Verknüpfung der dabei entstehenden freien Acylgruppe mit anderen Aminogruppen. Bei Proteinen, insbesondere bei der Gelatine, katalysiert die Transglutaminase somit in erster Linie eine Verknüpfung von Giutaminresten mit den ε-Aminogruppen von Lysinresten, d.h. die Bildung sowohl inter- als auch intramolekularer kovalenter Bindungen. Transglutaminase ist als natürliches Enzym physiologisch unbedenklich, sofern sie in entsprechend gereinigter Form zum Einsatz kommt.

Bevorzugt werden im Rahmen der Erfindung Transglutaminasen bakterieller Herkunft eingesetzt, die in hoher Qualität und Reinheit erhältlich sind. Es kann aber auch humane Transglutaminase eingesetzt werden, die insbesondere durch rekombinante Genexpression hergestellt werden kann.

Bevorzugt wird die Transglutaminase in immobilisierter Form auf einem Trägermaterial eingesetzt. Dadurch wird eine gleichmäßigere Verteilung der Enzymmoleküle im Kleber möglich, so dass eine höhere Aktivität bei gleicher Menge an Enzym erreicht werden kann. Bevorzugte Trägermaterialien für Transglutaminase sind Oligosaccharide.

Erfindungsgemäß erfolgt die Vernetzung der Gelatine im Applikationsbereich des Körpers, d.h. die Gelatine und das Vernetzungsmittel sollten erst nach, während oder unmittelbar vor der Verabreichung unter Bedingungen miteinander in Kontakt kommen, die das Ablaufen der Vernetzungsreaktion ermöglichen. Um dies zu gewährleisten, sind verschiedene Formen der Bereitstellung und der Verabreichung der Gelatine und des Vernetzungsmittels denkbar. Die oben bereits erwähnten grundlegenden Alternativen (i) und (ii) sollen im Folgenden näher beschrieben werden.

Gemäß der Variante (i) der Erfindung erfolgt die Anwendung des medizinischen Klebers dergestalt, dass die Gelatine und das Vernetzungsmittel zur Bildung eines vernetzenden medizinischen Klebers gemischt werden und dieser in den Applikationsbereich verabreicht wird. Bevorzugt handelt es sich bei einem solchen Kleber um eine wässrige Lösung, die das Vernetzungsmittel und die Gelatine gelöst enthält.

Bei dieser Vorgehensweise wird sichergestellt, dass eine homogene Verteilung beider Komponenten in der Lösung vorliegt. Eine solche Lösung kann auch auf einfache Weise verabreicht werden, insbesondere durch einfaches Auftragen oder Aufsprühen auf den Applikationsbereich oder durch Injektion. Allerdings sollte eine solche Lösung in der Regel erst unmittelbar vor der Verabreichung hergestellt werden, um zu vermeiden, dass die Vernetzungsreaktion vor dem Erreichen des Applikationsbereichs bereits zu weit fortgeschritten ist und die Viskosität der Lösung z.B. für eine Injektion zu hoch wird. Je nach Art der Gelatine und des Vernetzungsmittels ist aber auch denkbar, dass eine die beiden Komponenten enthaltende Lösung einige Zeit gelagert werden kann, insbesondere bei niedrigen Temperaturen, ohne das die Vernetzungsreaktion bereits in einem die Verabreichung beeinträchtigenden Ausmaß abläuft.

Bevorzugt wird die wässrige Lösung durch Auflösen einer Feststoffmischung, welche die Gelatine und das Vernetzungsmittel vorzugsweise in lyophilisierter Form umfasst, hergestellt. Diese Form der Bereitstellung ist insbesondere in dem Fall geeignet, wenn als Vernetzungsmittel Transglutaminase eingesetzt wird.

Das Bereitstellen von Gelatine und Vernetzungsmittel in dieser festen Form, in der die enzymatische Reaktion nicht ablaufen kann, hat den Vorteil, dass die Mischung eine relativ hohe Lagerstabilität aufweist. Gleichzeitig ist die Handhabung für den behandelnden Arzt einfach, da er nur eine einzige Feststoffmischung in einem flüssigen Medium auflösen muss.

Das Auflösen der Feststoffmischung sollte unmittelbar vor der Verabreichung der wässrigen Lösung erfolgen, d.h. insbesondere weniger als 10 Minuten, bevorzugt weniger als 5 Minuten vorher, bezogen auf die jeweils vorgegebene Temperatur im Applikationsbereich.

Durch das Vorliegen der Gelatine in lyophilisierter Form wird deren Löslichkeit auch bei niedrigeren Temperaturen deutlich verbessert. Dies ist von Bedeutung, weil eine Verabreichung des medizinischen Klebers in der Regel nicht oberhalb der Körpertemperatur von 37°C erfolgen sollte. Das Auflösen der Feststoffmischung erfolgt deshalb vorzugsweise bei einer Temperatur von 37°C oder weniger. Lyophilisierte Gelatine ist bei diesen Temperaturen, insbesondere bei Raumtemperatur, gut löslich, da sie zumindest überwiegend in amorpher Form vorliegt.

Im Hinblick auf die Geschwindigkeit der Bildung des Gelatinegels sowie dessen Festigkeit ist die eingesetzte Menge an Vernetzungsmittel im Verhältnis zu der Menge an Gelatine von entscheidender Bedeutung. In dem Fall, dass Transglutaminase verwendet wird, sind in der oben beschriebenen Mischung bevorzugt 0,6 bis 80 Units Transglutaminase pro Gramm Gelatine enthalten, weiter bevorzugt 10 bis 40 Units/g. Auf die Kinetik der Gelbildung, die sich unter anderem aus der Wahl dieses Verhältnisses ergibt, wird weiter unten im Detail eingegangen.

Im Hinblick auf die erste Variante (i) betrifft die vorliegende Erfindung somit auch eine Feststoffmischung, die Gelatine und Transglutaminase vorzugsweise in lyophilisierter Form umfasst.

Bei der oben genannten Variante (ii) der Erfindung erfolgt die Anwendung des medizinischen Klebers dergestalt, dass die Gelatine und das Vernetzungsmittel in voneinander getrennter Form bereitgestellt und gleichzeitig oder nacheinander unter Bildung des vernetzenden medizinischen Klebers verabreicht werden. Dabei kann eine Durchmischung der beiden Komponenten zu verschiedenen Zeitpunkten erfolgen, wie im Folgenden beschrieben wird.

Eine bevorzugte Form der Bereitstellung besteht darin, dass sowohl die Gelatine als auch das Vernetzungsmittel in Form von separaten wässrigen Lösungen bereitgestellt werden. Diese können dann vom behandelnden Arzt gemischt und in Form einer einzigen Lösung verabreicht werden, wie dies bereits oben beschrieben wurde. Das Mischen sollte dabei weniger als 10 Minuten, bevorzugt weniger als 5 Minuten vor der Verabreichung erfolgen.

Um einen zu frühen Beginn der Vernetzungsreaktion mit größerer Sicherheit auszuschließen, ist es jedoch bevorzugt, wenn die Gelatinelösung und die Vernetzungsmittellösung nicht vor, sondern erst während oder nach der Verabreichung miteinander in Kontakt kommen. Dies kann insbesondere durch eine gleichzeitige Verabreichung der beiden (getrennten) Lösungen erreicht werden.

Je nach Art der eingesetzten Mittel zur Verabreichung der Lösungen (z.B. eine oder mehrere Injektionskanülen oder andere Applikatoren) kann dabei die Durchmischung der gleichzeitig verabreichten Lösungen vor, beim oder nach dem Erreichen des Applikationsbereichs erfolgen. Eine möglichst frühzeitige Durchmischung, d.h. vor dem Erreichen des Applikationsbereichs, ist jedoch vorteilhaft, um eine hohe Homogenität der im Applikationsbereich ankommenden Lösung und damit die Ausbildung eines gleichmäßig vernetzten Gelatinegels zu gewährleisten.

Bei einer bevorzugten Ausführungsform der Erfindung erfolgt eine gleichzeitige Verabreichung der Gelatinelösung und der Vernetzungsmittellösung durch Injektion beider Lösungen mit einer Mehrkammerapplikationsvorrichtung, beispielsweise einer Doppelkammerspritze. Dabei befinden sich die Gelatine- und die Vernetzungsmittellösung in getrennten Kammern der Applikationsvorrichtung und werden beispielsweise durch eine gemeinsame Injektionskanüle an den gewünschten Applikationsbereich bereits vermischt verabreicht. Das Durchmischen der beiden Lösungen erfolgt somit bei der Verabreichung, beispielsweise beim Eintritt in die Kanüle. Um eine möglichst intensive Durchmischung zu bewirken, ist es bevorzugt, wenn die Mehrkammerapplikationsvorrichtung ein Mischelement umfasst. Dabei kann es sich insbesondere um eine geometrische Struktur (statischer Mischer) im Fließweg der Kanüle handeln, an der eine Durchmischung, insbesondere eine Verwirbelung der beiden Lösungen erfolgt.

Im Hinblick auf die zweite Variante (ii) betrifft die vorliegende Erfindung somit auch eine Mehrkammerapplikationsvorrichtung, welche eine wässrige Gelatinelösung und eine wässrige Vernetzungsmittellösung in getrennten Kammern enthält, wobei die Gelatine eine Viskosität von 7 mPa·s oder mehr aufweist, gemessen in einer 6,7 Gew-%igen Lösung bei 60°C.

Alternativ ist es auch möglich, die wässrige Gelatinelösung und die wässrige Vernetzungsmittellösung nacheinander in den Applikationsbereich zu verabreichen. Auch in diesem Fall ist sichergestellt, dass die Vernetzung der Gelatine erst im Applikationsbereich stattfindet.

Bei einer weiteren bevorzugten Ausführungsform der Erfindung wird einerseits eine wässrige Gelatinelösung bereitgestellt sowie ein Vernetzungsmittel in fester Form. Diese Variante ist insbesondere im Fall von enzymatischen Vemetzungsmitteln wie Transglutaminase geeignet, deren Haltbarkeit in dieser Form in der Regel höher ist als in Lösung. Das Enzym kann insbesondere in Form eines lyophilisierten Pulvers bereitgestellt werden, das dann vor dem Verabreichen in die Gelatinelösung zudosiert und nachfolgend aufgelöst wird.

Aus den bereits oben genannten Gründen ist es bevorzugt, wenn die Verabreichung der wässrigen Gelatinelösung bei einer Temperatur von 37°C oder weniger erfolgt. Die Herstellung der Gelatinelösung kann jedoch auch bei höheren Temperaturen, z.B. bei 60°C, erfolgen.

Wird die Lösung dann bei Raumtemperatur oder unter Kühlung gelagert, kann die Gelatine zwar gelieren und sich verfestigen, sie kann dann aber durch Erwärmen auf bis zu 37°C unmittelbar vor der Verabreichung wieder in Lösung gebracht werden.

Die Konzentration der verabreichten Gelatinelösung ist bevorzugt so gewählt, dass die Gelatinekonzentration in dem medizinischen Kleber 5 bis 20 Gew.% beträgt. Es wurde gefunden, dass niedrigere Gelatinekonzentrationen in der Regel nicht zu gut vernetzten Gelatinegelen mit einer ausreichenden Festigkeit führen.

Im Fall der Verwendung von Transglutaminase als Vernetzungsmittel ist deren Menge und Konzentration in einer Transglutaminaselösung vorzugsweise so gewählt, dass sich, wie bereits im Zusammenhang mit der Variante (i) beschrieben, eine Menge von 0,6 bis 80 Units Transglutaminase pro Gramm Gelatine in dem medizinischen Kleber ergibt. Weiter bevorzugt ist ein Verhältnis von 10 bis 40 Units/g. Dabei kann das Volumen der Transglutaminaselösung in der Regel deutlich kleiner gewählt werden als das der Gelatinelösung, so dass letztere durch das Mischen mit der Transglutaminaselösung nicht wesentlich verdünnt wird.

Die Geschwindigkeit der Vernetzungsreaktion sowie die Festigkeit des gebildeten Gelatinegels hängen in hohem Maße von der Gelatinekonzentration in dem medizinischen Kleber und von dem Verhältnis zwischen Gelatine und Vernetzungsmittel ab. Diese Parameter können innerhalb der oben genannten bevorzugten Bereiche variiert werden, um den Einfluss weiterer Faktoren auszugleichen.

Solche Faktoren sind z.B. die Art der eingesetzten Gelatine, insbesondere deren Viskosität und mittleres Molekulargewicht, sowie die Art des Vernetzungsmittels, insbesondere im Fall von Transglutaminase auch deren Art und Herkunft.

Die Kinetik und das Ausmaß der Vernetzungsreaktion können durch verschiedene physikalische Parameter beschrieben werden. Um diese zu messen, wird die Bildung des Gelatinegels, wie sie bei der therapeutischen Anwendung *in vivo* abläuft, durch eine entsprechende Reaktion *in vitro* nachgestellt. Der Beginn der Vernetzungsreaktion ist dabei jeweils durch den Zeitpunkt definiert, in dem die Gelatine und das Vernetzungsmittel in einer wässrigen Lösung miteinander in Kontakt kommen.

Die Geschwindigkeit der Bildung des vernetzten Gelatinegels lässt sich insbesondere durch die Angabe des sogenannten Gelpunktes charakterisieren. Der Gelpunkt ist dabei als derjenige Zeitpunkt nach dem Beginn der Vernetzungsreaktion definiert, an dem der Speichermodul G' und der Verlustmodul G" des Gelatinegels gleich groß sind (siehe auch T. Metzger, Das Rheologie-Handbuch, Verlag Vincentz, 2000, Seite 173 ff).

Bei einer unvernetzten, flüssigen Gelatinelösung liegt G' deutlich unterhalb von G". Im Verlauf der Vernetzungsreaktion, d.h. mit zunehmender Gelbildung, steigen sowohl der Speicher- als auch der Verlustmodul an, wobei G' stärker ansteigt als G". Der oben genannte Gelpunkt lässt sich somit aus dem Schnittpunkt der beiden Kurven in einem Diagramm, in dem G' und G" über der Zeit aufgetragen sind, ermitteln. Experimentell lässt sich der Gelpunkt auch als der Zeitpunkt ermitteln, an dem im Verlauf der Vernetzungsreaktion erstmals eine Gelfestigkeit (siehe unten) gemessen werden kann.

Für die Verwendung gemäß der vorliegenden Erfindung ist es bevorzugt, wenn der Gelpunkt des vernetzten Gelatinegels 10 Minuten oder weniger nach Beginn der Vernetzungsreaktion erreicht wird, besonders bevorzugt 5 Minuten oder weniger nach Beginn der Vernetzungsreaktion. Die vorgenannten Zeiten beziehen sich jeweils auf die vorgegebene Temperatur des Applikationsbereiches. Der Gelpunkt kann je nach Anwendungsbereich des medizinischen Klebers durch die Wahl der oben genannten Parameter beeinflusst werden.

Wie bereits angesprochen wurde, läuft die Vernetzung der Gelatine kontrolliert ab, d.h. die Gelfestigkeit steigt kontinuierlich an und erreicht erst eine bestimmte Zeit nach dem Gelpunkt ihren maximalen Wert. Auch nach Erreichen des Gelpunktes ist der medizinische Kleber zum Teil noch plastisch verformbar und kann an die Struktur des Applikationsbereiches angepasst werden. In manchen Fällen ist eine sehr rasche Gelbildung erwünscht, z.B. wenn bei Operationen durch Verkleben von Blutgefäßen eine Blutung gestillt werden muss. Erfolgt die Gelbildung allerdings zu schnell, so besteht die Gefahr, dass der medizinische Kleber seine Fließfähigkeit zu früh verliert und dem behandelnden Arzt nicht genügend Zeit für die Verabreichung zur Verfügung steht.

Im Hinblick auf die mechanischen Eigenschaften des vernetzten Gelatinegels ist es bevorzugt, wenn dieses eine Gelfestigkeit von 100 g oder mehr aufweist, gemessen mit einem Stempel mit einem Durchmesser von 12,7 mm bei einer Eindringtiefe von 4 mm. Diese Angaben beziehen sich auf das Eindrücken eines kreisförmigen Stempels mit einem Durchmesser von 12,7 mm in das Gelatinegel senkrecht zu dessen Oberfläche, wobei der Stempel aus Polymethylmethacrylat besteht und eine polierte Oberfläche aufweist (siehe "Standardised Methods for the Testing of Edible Gelatine", Gelatine Monograph, Juni 2005, GME).

Die Gelfestigkeit kann auch als Kraft ausgedrückt werden: Bei einer Gelfestigkeit von 100 g sind 0,981 N erforderlich, um den Stempel 4 mm tief in das Gelatinegel einzudrücken. Bezogen auf die Stempelfläche beträgt die Gelfestigkeit in diesem Fall 774 mN/cm².

Die beschriebene Gelfestigkeit bezieht sich auf den maximalen Wert, der bei dem vernetzten Gelatinegel erreicht wird. Ein wichtiger Parameter ist in diesem Zusammenhang jedoch auch der Anstieg der Gelfestigkeit pro Zeiteinheit, der ebenfalls durch die Wahl der Gelatinekonzentration, der Menge an Vernetzungsmittel usw. beeinflusst werden kann. Bevorzugt liegt der Anstieg der Gelfestigkeit des vernetzten Gelatinegels in den ersten 10 min nach Erreichen des Gelpunktes im Bereich von 5 bis 200 mN/cm²·min, insbesondere bei 30 bis 150 mN/cm²·min.

Als Maß für die Klebrigkeit des vernetzten Gelatinegels kann dessen Haftung an einer glatten Kunststoffoberfläche, z.B. aus poliertem Polymethylmethacrylat, herangezogen werden, welche bevorzugt 200 mN/cm² oder mehr beträgt.

Es wurde bereits angesprochen, dass neben anderen Faktoren auch die Viskosität der eingesetzten Gelatine einen Einfluss auf die Gelbildung ausübt, wobei eine höhere Viskosität in der Regel mit einer schnelleren Gelbildung einhergeht. Unter der Viskosität der Gelatine ist in diesem Zusammenhang die Viskosität einer 6,7 Gew.%igen Standardlösung der Gelatine in Wasser bei 60°C zu verstehen. Diese beträgt 7 mPa·s oder mehr für die im Rahmen der vorliegenden Erfindung eingesetzte Gelatine.

Die Viskosität von Gelatine ist sowohl von deren Herkunft als auch vom jeweiligen Herstellungsverfahren abhängig und kann durch bestimmte Maßnahmen weiter beeinflusst werde.

Bei einer bevorzugten Ausführungsform der Erfindung wird eine Gelatine eingesetzt, die zuvor einer thermischen Vorbehandlung bei vermindertem Druck unterworfen wurde. Durch eine solche Vorbehandlung lässt sich die Viskosität der Gelatine steigern, wobei dieser Effekt in erster Linie auf eine thermische Wasserabspaltung innerhalb der Gelatinemoleküle zurückzuführen ist.

Die thermische Vorbehandlung wird bevorzugt bei Temperaturen von 80 bis 160°C durchgeführt, da unterhalb von 80°C die beobachteten Effekte relativ schwach ausgeprägt sind und oberhalb von 160°C eine unerwünschte Verfärbung der Gelatine auftreten kann. Am meisten bevorzugt sind Werte im Bereich von 90 bis 120°C.

Die Gelbildung ist ferner auch vom Molekulargewicht der Gelatine abhängig. Die Verwendung von Gelatine mit einem hohen mittleren Molekulargewicht, insbesondere von 140 kDa oder mehr, ist dabei bevorzugt, da in diesem Fall bereits durch eine geringere Anzahl an Vernetzungsstellen ein unlösliches Gelatinegel entsteht als bei einer Gelatine mit niedrigerem Molekulargewicht.

Alternativ oder zusätzlich zu einer gezielten Auswahl oder Modifikation der eingesetzten Gelatine können die Eigenschaften des erfindungsgemäßen medizinischen Klebers auch dadurch beeinflusst werden, dass zwei oder mehr Gelatinen mit unterschiedlichen Viskositäten und/oder Bloom-Werten miteinander gemischt werden. Beispielsweise kann durch verschiedene Mischungsverhältnisse von hochviskoser Knochengelatine mit einer niedrigviskosen Fischgelatine die Geschwindigkeit der Gelbildung über einen weiten Bereich variiert werden.

Bei bestimmten Anwendungen kann es erwünscht sein, dass der medizinische Kleber bzw. die die Gelatine enthaltende Lösung bereits vor dem Beginn der Vernetzungsreaktion eine relativ hohe Viskosität aufweist. Dies ist zum Beispiel dann der Fall, wenn im Applikationsbereich ein Druck auf den medizinischen Kleber ausgeübt wird, so dass eine Lösung mit zu geringer Viskosität sofort wieder aus dem Applikationsbereich herausgepresst würde.

Eine Erhöhung der Viskosität kann vorteilhafterweise dadurch erreicht werden, dass der medizinische Kleber ein viskositätserhöhendes Polymer umfasst. Ein bevorzugtes viskositätserhöhendes Polymer ist beispielsweise Carboxymethylzellulose. Durch dessen Zusatz kann die Viskosität einer Gelatinelösung um das 20fache und mehr gesteigert werden.

Bei einer weiteren bevorzugten Ausführungsform der Erfindung wird für die Herstellung des medizinischen Klebers eine partiell vernetzte Gelatine eingesetzt, d.h. die Gelatine wird vor der erfindungsgemäßen Verabreichung bereits einem ersten (partiellen) Vernetzungsschritt unterzogen. Die partiell vernetzte Gelatine kann, wie oben beschrieben, mit dem Vernetzungsmittel gemischt oder mit diesem gleichzeitig oder aufeinander folgend verabreicht werden, wobei die Bildung des vernetzten Gelatinegels im Applikationsbereich dann einen zweiten Vernetzungsschritt darstellt.

Durch die Verwendung von partiell vernetzter Gelatine kann einerseits die Viskosität der zu verabreichenden Gelatinelösung deutlich erhöht werden, was mit den oben erwähnten Vorteilen einhergeht. Des Weiteren kann durch diese Maßnahme auch eine wesentlich schnellere Gelbildung erreicht werden, wobei Gelpunkte deutlich unterhalb von 5 Minuten, insbesondere im Bereich von wenigen Sekunden, realisiert werden können. Eine sehr schnelle Gelbildung, die fast unmittelbar nach der Applikation des medizinischen Klebers einsetzt, kann bei bestimmten Anwendungen vorteilhaft sein, wenn jegliches unerwünschtes Wegfließen des Klebers aus dem Applikationsbereich vermieden werden soll. Auch bei Verwendung von partiell vernetzter Gelatine bleibt jedoch der Vorteil der kontrollierten Vernetzung im Applikationsbereich erhalten, d.h. auch wenn die Gelbildung sehr schnell erfolgt, steigt die Gelfestigkeit danach kontinuierlich an und erreicht erst eine bestimmte Zeit nach dem Gelpunkt ihren maximalen Wert.

Damit die Lösung der partiell vernetzten Gelatine zwar hochviskos, aber unter den Applikationsbedingungen immer noch fließfähig ist, sollte der Grad der partiellen Vernetzung nicht zu hoch sein. Dieser kann durch die Bedingungen, unter denen die partiell vernetzte Gelatine hergestellt wird, kontrolliert werden, insbesondere durch die Gelatinekonzentration, die Menge des Vernetzungsmittels und die Dauer der partiellen Vernetzungsreaktion. Bevorzugt ist die eingesetzte Gelatine unter Verwendung von Transglutaminase partiell vernetzt. Neben den bereits oben erwähnten Vorteilen bietet die Verwendung von Transglutaminase die Möglichkeit, die partielle Vernetzungsreaktion durch die Inaktivierung des Enzyms nach einer definierten Reaktionszeit abzubrechen, insbesondere durch eine thermische Denaturierung oder ein Oxidationsmittel wie z.B. Wasserstoffperoxid.

Wenn die partielle Vernetzung der Gelatine mittels Transglutaminase erfolgt, so können hierfür deutlich geringere Mengen an Transglutaminase im Verhältnis zur Gelatine eingesetzt werden, als dies im Rahmen der Verabreichung des medizinischen Klebers der Fall ist. Vorzugsweise ist die Gelatine unter Verwendung von weniger als 10 Units Transglutaminase pro Gramm Gelatine partiell vernetzt, insbesondere unter Verwendung von 1 bis 3 Units Transglutaminase pro Gramm Gelatine.

Bei einigen Anwendungen kann es vorteilhaft sein, wenn der medizinische Kleber einen oder mehrere therapeutische Wirkstoffe umfasst, z.B. entzündungshemmende und/oder schmerzstillende Mittel, antibiotisch wirkende Substanzen sowie Faktoren, die die Wundheilung und/oder die Angiogenese fördern.

Um eine verzögerte und/oder kontinuierliche Freisetzung zu ermöglichen, ist es bevorzugt, wenn der oder die Wirkstoffe in verkapselter Form in dem medizinischen Kleber vorliegen. Insbesondere können Wirkstoffe in Gelatinekügelchen verkapselt werden.

Bei einer weiteren vorteilhaften Ausführungsform des medizinischen Klebers umfasst dieser einen Farbstoff. Dadurch kann der behandelnde Arzt bei der Verabreichung genau erkennen, an welcher Stelle sich der Kleber innerhalb des Applikationsbereichs befindet und wie viel Kleber bereits verabreicht worden ist. Als Farbstoff kann beispielsweise Methylenblau eingesetzt werden, welches körperverträglich ist und sich vom Gewebe deutlich abhebt.

Im Folgenden sollen einige bevorzugte Anwendungsbereiche des erfindungsgemäßen medizinischen Klebers dargestellt werden.

Eine bevorzugte Ausführungsform der Erfindung betrifft die Verwendung von Gelatine und einem Vernetzungsmittel zur Herstellung eines vernetzenden medizinischen Klebers zum Stillen von Blutungen, wobei der medizinische Kleber auf einen von einer Blutung betroffenen Applikationsbereich aufgebracht wird, so dass durch die Bildung des vernetzten Gelatinegels die Blutung gestillt wird.

Das Aufbringen auf den Applikationsbereich kann gemäß einer der oben beschriebenen Varianten erfolgen, wobei bevorzugt eine die Gelatine und das Vernetzungsmittel enthaltende wässrige Lösung aufgegossen oder aufgesprüht wird. Die Lösung kann sich gleichmäßig verteilen und den betroffenen Bereich vollständig abdecken. Das sich bildende vernetzte Gelatinegel bildet eine Schutzschicht nach außen. Dabei werden beschädigte oder durchtrennte Blutgefäße, die sich in dem Applikationsbereich befinden, durch das Gelatinegel verschlossen bzw. verklebt.

Insbesondere betrifft die Erfindung derartige Anwendungen bei Operationen. Der Einsatz des erfindungsgemäßen medizinischen Klebers bietet besondere Vorteile, wenn Eingriffe an besonders stark durchbluteten Geweben oder Organen, wie z.B. der Leber oder dem Knochen, durchgeführt werden. Unter Anwendung herkömmlicher Methoden müsste dabei eine Vielzahl von Blutgefäßen von Hand abgeklemmt werden, was sehr umständlich, zeitaufwändig und bei großflächigen, diffusen Blutungen häufig auch unmöglich ist. Im Gegensatz dazu kann die Blutung unter Anwendung des erfindungsgemäßen medizinischen Klebers sehr schnell und einfach gestillt werden, indem die Schnittfläche an dem Gewebe bzw. dem Organ mit einem vernetzten Gelatinegel überzogen und somit verklebt wird.

Gegenstand der Erfindung ist in diesem Zusammenhang auch ein Verfahren zum Stillen von Blutungen beim Menschen oder beim Tier, insbesondere bei Operationen, wobei das Verfahren das Verabreichen von Gelatine und einem Vernetzungsmittel, getrennt oder bereits vor Erreichen des Applikationsbereichs gemischt, umfasst.

Die Erfindung betrifft ferner die Verwendung von Gelatine und einem Vernetzungsmittel zur Herstellung eines vernetzenden medizinischen Klebers zur Behandlung von Verletzungen und/oder Verbrennungen der menschlichen oder tierischen Haut. Bei einer solchen äußerlichen Anwendung kann sowohl eine blutungsstillende als auch eine reine Schutzfunktion des medizinischen Klebers im Vordergrund stehen. Durch die guten Klebeeigenschaften können die betroffenen Hautbereiche zuverlässig abgedeckt werden.

Ein weiterer Anwendungsbereich der Erfindung betrifft die Verwendung des medizinischen Klebers zur Refixierung bzw. Fixierung von autologen (z.B. Knorpel- oder Knochenfragmente) oder allogenen Geweben (z.B. Spongiosaplastik aus homologem Knochen) oder von unterschiedlichen Implantaten in einem Applikationsbereich des menschlichen oder tierischen Körpers. Auf Grund der guten Klebeeigenschaften des vernetzten Gelatinegels können damit Implantate verschiedener Art für einen bestimmten Zeitraum, d.h. bis zur Resorption des Klebers, im oder am Gewebe fixiert werden.

Ferner kann es sich bei den zu fixierenden Implantaten um resorbierbare Materialien handeln. Hier sind insbesondere auch solche Materialien zu nennen, die entweder zuvor *in vitro* mit lebenden Zellen besiedelt wurden oder in die nach ihrer Implantation *in vivo* Zellen einwachsen. In diesen Fällen unterliegen sowohl das Bio- bzw. Trägermaterial als auch der Kleber einem biologischen Abbau.

Auch künstliche Implantate, die zumindest teilweise aus Kunststoff und/oder Metall bestehen, können mit Hilfe des erfindungsgemäßen Klebers in einem Applikationsbereich des Körpers fixiert werden. Ein solches Einkleben von Implantaten kann für eine bestimmte Zeit erforderlich sein, bis das umgebende Gewebe eine ausreichende Stützfunktion für das Implantat übernehmen kann.

Der erfindungsgemäße Kleber kann auch zum Fixieren und/oder Verschließen von Nervenleitschienen eingesetzt werden. Dabei handelt es sich um röhrchenförmige Implantate, die als Leitstruktur für das Zusammenwachsen durchtrennter Nervenbahnen dienen. Im Innenraum einer Nervenleitschiene wachsen jeweils einzelne Nervenzellen (Axone), wobei deren Enden manuell in das Röhrchen eingeführt und dort fixiert werden müssen. Dies kann auf vorteilhafte Weise durch Verkleben des Axons in der Nervenleitschiene mittels des erfindungsgemäßen medizinischen Klebers geschehen. Bei dieser Anwendung ist es besonders vorteilhaft, die Viskosität des Klebers durch Zusatz von z.B. Carboxymethylcellulose zu erhöhen, um sicherzustellen, dass das Axon auch in noch unvernetztem Zustand in dem Röhrchen verbleibt.

Gemäß einer weiteren Anwendung des erfindungsgemäßen Klebers wird dieser zum Abdichten von chirurgischen Nähten eingesetzt. Durch die guten Klebeeigenschaften des vernetzten Gelatinegels können Nähte effektiv abgedichtet werden, um unerwünschte Durchtrittsöffnungen zwischen verschiedenen Geweben oder Organen zu vermeiden. Zusätzlich wird die Naht vor äußeren Beanspruchungen geschützt.

Der erfindungsgemäße Kleber kann auch für die Abdichtung knöcherner Bohrkanäle, z.B. beim Kreuzbandersatz, angewendet werden. Hierdurch wird zum Einen die Blutung gestoppt, und zum Anderen wird durch die Abdichtung das Eindringen von Synovialflüssigkeit zwischen Implantat und Knochenwand verhindert. Das Eindringen von Synovialflüssigkeit in den Bohrkanal kann nach dem derzeitigen Kenntnisstand zu einem schlechten Einwachsen des Implantats in den Knochen führen, woraus sich im weiteren Verlauf ein Transplantatversagen ergeben kann.

Die Erfindung betrifft des Weiteren auch die Verwendung des medizinischen Klebers als Trennschicht für Organe oder Gewebe. Eine solche separierende Wirkung des Klebers kann wünschenswert bzw. erforderlich sein, um beispielsweise während oder nach chirurgischen Eingriffen einen unerwünschten direkten Kontakt zwischen verschiedenen Organen und/oder Geweben zu verhindern. Dabei kann aufgrund der hohen Klebkraft des erfindungsgemäßen Klebers gleichzeitig eine Fixierung der beteiligten Gewebe erfolgen, so dass dieser hier eine doppelte Funktion erfüllt.

Bei vielen Anwendungen des erfindungsgemäßen medizinischen Klebers ergibt sich ein zusätzlicher vorteilhafter Effekt durch die Angiogenese fördernde Wirkung von Gelatine. Es wurde gefunden, dass in Gegenwart von Gelatine, insbesondere auch in vernetzter Form, die Neubildung von Blutgefäßen im Gewebe stimuliert wird. Ein solcher Effekt ist beispielsweise bei der Anwendung des Klebers zur Behandlung von Wunden oder knöchernen Defekten, aber auch in vielen anderen Fällen, äußerst vorteilhaft, da er zur Heilung und/oder Regeneration des betroffenen Gewebes beiträgt.

Diese und weitere Vorteile der vorliegenden Erfindung werden anhand der nachfolgenden Beispiele unter Bezugnahme auf die Figuren näher erläutert. Es zeigen:
- Fig. 1A bis 1E:: Diagramme, in denen die Gelfestigkeit und Klebrigkeit eines erfindungsgemäßen vernetzten Gelatinegels für Gelatinen mit verschiedenen Viskositäten in Abhängigkeit von der Re- aktionszeit aufgetragen sind; und
- Fig. 2:: Diagramm, in dem die Gelfestigkeit und Klebrigkeit einer herkömmlichen auf Thrombin und Fibrinogen basierenden Zusammensetzung in Abhängigkeit von der Reaktionszeit aufgetragen sind.

### Beispiel

### Beispiel 1: Vernetzung von Gelatine mit Transglutaminase: Einfluss der Viskosität der Gelatine

Als Modellsystem für die medizinische Anwendung der erfindungsgemäßen Klebers wurde die Vernetzung von Gelatine mit dem enzymatischen Vernetzungsmittel Transglutaminase *in vitro* durchgeführt und die Kinetik der Bildung eines vernetzten Gelatinegels bestimmt.

### Herstellung einer Transglutaminase-Stammlösung

Für dieses und die nachfolgend beschriebenen Beispiele wurde eine rekombinante Transglutaminase aus humanen Keratinozyten eingesetzt.

Eine Stammlösung der Transglutaminase mit einer Konzentration von 30 Units/ml wurde hergestellt, indem die entsprechende Menge des Enzyms in destilliertem Wasser bei Raumtemperatur aufgelöst wurde. Die Lösung wurde sterilfiltriert, in Portionen von je 1,5 ml mit Hilfe von flüssigem Stickstoff eingefroren und bei ca. -18°C gelagert.

### Thermische Vorbehandlung von Gelatine bei vermindertem Druck

Für die Vernetzung mit Transglutaminase wurden Schweineschwartengelatinen mit unterschiedlichen Viskositäten gemäß der folgenden Tabelle 1 eingesetzt. Die Angabe der Viskosität bezieht sich dabei stets auf die Viskosität einer 6,7 Gew.%igen wässrigen Lösung der Gelatine bei 60°C.

**Tabelle 1**

| Bezeichnung | Viskosität (mPa·s) |
|---|---|
| Gelatine A | 3,73 (Vergleichsbsp.) |
| Gelatine B | 5,83 (Vergleichsbsp.) |
| Gelatine C | 7,62 |
| Gelatine D | 8,65 |

Die hochviskosen Gelatinen C und D wurden jeweils durch eine thermische Vorbehandlung von Gelatinen mit geringerer Viskosität hergestellt. Dabei wurde die Gelatine C durch thermische Vorbehandlung der Gelatine B erhalten sowie die Gelatine D durch thermische Vorbehandlung einer weiteren Schweineschwartengelatine mit einer Viskosität von 6,41 mPa·s.

Die thermische Vorbehandlung der Gelatine bei vermindertem Druck wurde so durchgeführt, dass jeweils ca. 700 g Gelatine in gemahlener Form mit Hilfe eines Rotationsverdampfers für 4 Stunden bei 105°C unter einem Vakuum von ca. 14 mbar gehalten wurden. Anschließend wurde die Gelatine über Nacht in einem geschlossenen Gefäß abkühlen gelassen.

### Durchführung der Vernetzungsreaktion

Für jede der vier Gelatinen A, B, C und D wurde eine 10 Gew.%ige Lösung der Gelatine in einer Mischung aus 30 Vol.% PBS-Puffer (pH 7,2) und 70 Vol.% destilliertem Wasser hergestellt. Hierfür wurde die Gelatine bei 60°C gelöst und die resultierende homogene Lösung auf 37°C temperiert.

Sämtliche Vernetzungsreaktionen wurden bei einer konstanten Temperatur von 37°C durchgeführt, um die bei der medizinischen Anwendung herrschenden Bedingungen möglichst gut anzunähern. Für jeden Ansatz wurden 5 ml der 10 Gew.%igen Gelatinelösung in ein zylindrisches Gefäß mit einem Durchmesser von 3 cm gegeben, das mit Hilfe eines Aluminiumblockes auf 37°C temperiert wurde. Die Vernetzungsreaktion wurde gestartet, indem 0,3 ml der Transglutaminase-Stammlösung (30 Units/ml) und 0,9 ml destilliertes Wasser, jeweils auf 37°C vorgewärmt, hinzugegeben wurden und die resultierende Reaktionsmischung sofort durchmischt wurde. Dies entspricht einer auf die Gelatine bezogenen Enzymmenge von 18 Units/g.

### Bestimmung der Gelfestigkeit und Klebrigkeit in Abhängigkeit von der Reaktionszeit

Während des Verlaufs der Vernetzungsreaktion wurden in Abständen von 50 s mittels einer Kraft/Weg-Messvorrichtung vom Typ Zwick BZ 2.5/TN1S (Hersteller: Zwick GmbH & Co. KG, Ulm) die Gelfestigkeit und die Klebrigkeit der Reaktionsmischung bestimmt.

Die Bestimmung läuft so ab, dass bei jedem Messzyklus, d.h. alle 50 s, ein kreisförmiger Stempel mit einem Durchmesser von 12,7 mm senkrecht zur Oberfläche der Reaktionsmischung 4 mm tief in diese eingetaucht bzw. eingedrückt und die hierfür erforderliche Kraft gemessen wird. Anschließend wird der Stempel, der eine polierte Polymethylmethacrylatoberfläche aufweist, wieder nach oben gezogen. Sofern bereits ein vernetztes Gelatinegel vorliegt, haftet dieses beim Herausziehen des Stempels an diesem an. Die erforderliche Kraft, um den Stempel soweit nach oben zu ziehen, dass sich das Gelatinegel ablöst, wird ebenfalls gemessen.

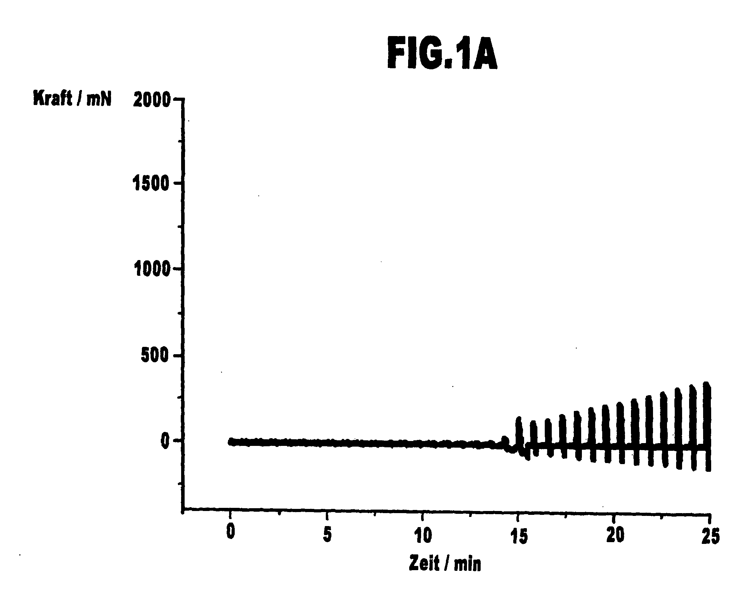

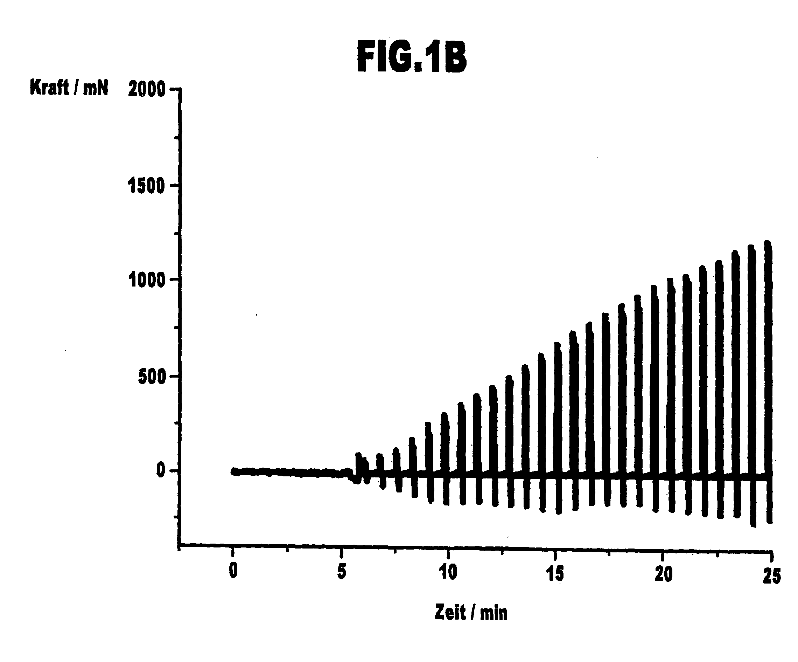

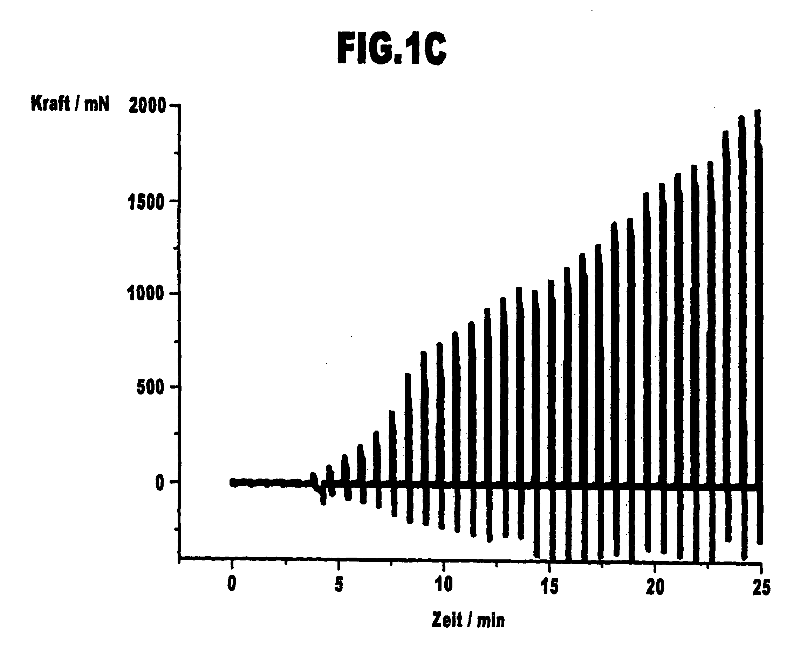

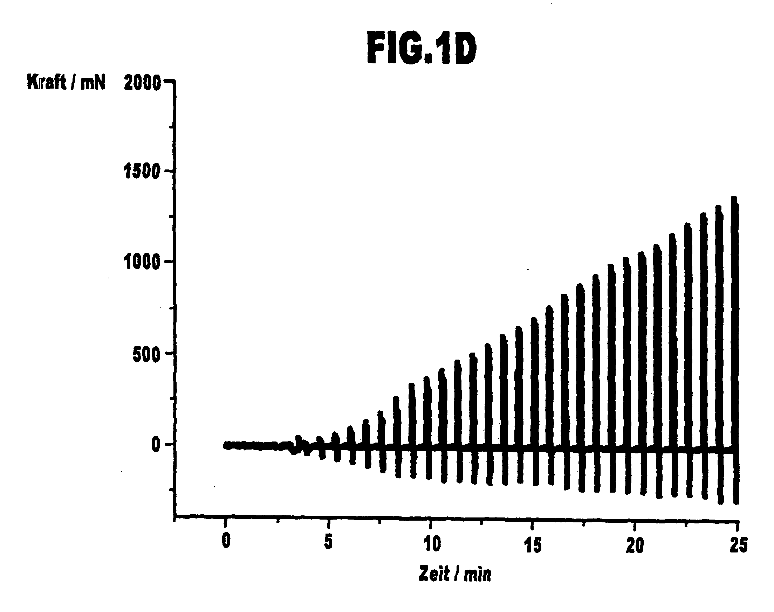

In den Figuren 1A bis 1D ist die gemessene Kraft in Abhängigkeit von der Reaktionszeit (Beginn der Vernetzungsreaktion bei 0 min) für die vier Ansätze mit den Gelatinen A, B, C bzw. D aufgetragen. Die positiven Kraftwerte geben die für das Eindrücken des Stempels erforderliche Kraft an, d.h. die Gelfestigkeit (981 mN entsprechen einer Gelfestigkeit von 100 g). Die negativen Kraftwerte geben die Klebrigkeit (Haftung) des Gelatinegels an, d.h. die zum Herausziehen des Stempels bis zur Ablösung des Gelatinegels erforderliche Kraft.

Während einer Anfangsphase der Reaktion liegen sowohl die Gelfestigkeit als auch die Klebrigkeit im Wesentlichen bei null, d.h. es liegt eine fließfähige Lösung vor. Bei der medizinischen Anwendung entspricht dies dem Zeitraum, in dem die Mischung in den Applikationsbereich des Körpers verabreicht werden kann. Der früheste Zeitpunkt, an dem eine merklich von null verschiedene Gelfestigkeit gemessen werden kann, ist der Gelpunkt. Zu diesem Zeitpunkt sind der Speichermodul G' und der Verlustmodul G" gleich groß.

Die verschiedenen Gelpunkte der Ansätze A bis D sind in der folgenden Tabelle 2 aufgeführt, wobei die Werte jeweils aus drei Experimenten gemittelt sind. Es sind jeweils die auf die Fläche bezogene Gelfestigkeit und Haftung des Gelatinegels angegeben, welche 10 min nach dem Gelpunkt gemessen wurden (die Stempelfläche beträgt 1,267 cm²). Diese Angaben dienen in erster Linie dem Vergleich der verschiedenen Ansätze untereinander; es wird aus den Figuren 1A bis 1D deutlich, dass insbesondere bei der Gelfestigkeit im weiteren Verlauf der Vernetzungsreaktion zum Teil noch deutlich höhere Werte erreicht werden.

**Tabelle 2**

| Ansatz | Viskosität der Gelatine | Gelpunkt | Gelfestigkeit nach 10 min | Haftung nach 10 min |
|---|---|---|---|---|
| Gelatine A (vergleichs bsp.) | 3,73 mPa·s | 14 min | 281 mN/cm² | 103 mN/cm² |
| Gelatine B (vergleichs bsp.) | 5,83 mPa·s | 5 min | 536 mN/cm² | 167 mN/cm² |
| Gelatine C | 7,62 mPa·s | 3,3 min | 837 mN/cm² | 225 mN/cm² |
| Gelatine D | 8,65 mPa·s | 2,3 min | nicht gemessen | nicht gemessen |

Es zeigt sich, dass der Gelpunkt mit der Viskosität der eingesetzten Gelatine korreliert, d.h. bei einer hochviskosen Gelatine erfolgt die Gelbildung bei gleicher Menge an Vernetzungsmittel wesentlich schneller als bei einer niedrigviskosen. * (Vergleichsbsp.)

Nach Erreichen des Gelpunktes steigt die Gelfestigkeit bei allen vier Ansätzen kontinuierlich und im Wesentlichen linear an. Die Rate des Anstiegs ergibt sich aus der in Tabelle 2 angegebenen Gelfestigkeit nach 10 min, d.h. sie beträgt für die Gelatine A beispielsweise 28,1 mN/cm²·min. Die Tatsache, dass die maximale Gelfestigkeit erst allmählich erreicht wird, ist für die Anwendung des medizinischen Klebers von erheblichem Vorteil. Durch die kontrolliert ablaufende Vernetzung kann der Kleber auch nach der Verabreichung noch verteilt, plastisch verformt und an die jeweiligen Gewebestrukturen angepasst werden.

Bei den Ansätzen A, B und C korreliert die nach 10 min gemessene Gelfestigkeit und Haftung ebenfalls mit der Viskosität der eingesetzten Gelatine, so wie dies auch für den Gelpunkt der Fall ist. Bei der Gelatine D, deren Viskosität noch etwas höher ist als diejenige der Gelatine C, wird der Gelpunkt zwar sehr schnell erreicht, die Gelfestigkeit und Haftung nach 10 min sind jedoch geringer.

### Beispiel 2: Vernetzung von Gelatine mit Transglutaminase: Einfluss der Gelatinekonzentration

In diesem Beispiel wurde die thermisch vorbehandelte Gelatine C aus dem Beispiel 1 bei unterschiedlichen Gelatinekonzentrationen mit Transglutaminase vernetzt. Das Ansetzen der Reaktionsmischungen und die Messung der Gelfestigkeit erfolgten wie in Beispiel 1 beschrieben.

Die Konzentration der eingesetzten Gelatinelösungen, die Zusammensetzung der Reaktionsmischungen und die sich aus der Gelfestigkeitsmessung ergebenden Gelpunkte sind in der folgenden Tabelle 3 dargestellt. Ferner sind die auf die Fläche bezogene Gelfestigkeit und Haftung 10 min nach dem Gelpunkt angegeben.

**Tabelle 3**

| Ansatz | 2-1 | 2-2 | 2-3 | 2-4 |
|---|---|---|---|---|
| Konzentration der Gelatinelösung | 5 Gew.% | 8 Gew.% | 10 Gew.% | 12,7 Gew.% |
| Gelatinelösung | 5,9 ml | 5,9 ml | 5 ml | 5,9 ml |
| Transglutaminase-Stammlösung | 0,2 ml | 0,3 ml | 0,3 ml | 0,5 ml |
| destilliertes Wasser | - | - | 0,9 ml | - |
| Transglutaminase pro g Gelatine | 20,3 Units/g | 19,1 Units/g | 18,0 Units/g | 20 Units/g |
| Gelpunkt | 5,0 min | 4,0 min | 3,3 min | 2,0 min |
| Gelfestigkeit nach 10 min | 265 mN/cm² | 587 mN/cm² | 837 mN/cm² | 869 mN/cm² |
| Haftung nach 10 min | 79 mN/cm² | 137 mN/cm² | 225 mN/cm² | 300 mN/cm² |

Die Ergebnisse zeigen, dass durch eine Erhöhung der Gelatinekonzentration die Gelbildung beschleunigt werden kann. Der Gelpunkt wird früher erreicht, und die nach einer bestimmten Zeit erzielte Gelfestigkeit und Haftung sind höher. Bei den durchgeführten Versuchen ist dieser Trend deutlich erkennbar, obwohl bei den höheren Gelatinekonzentrationen die Menge an Vernetzungsmittel im Verhältnis zur Gelatine teils geringer war.

Die Wahl der Gelatinekonzentration stellt damit eine Möglichkeit dar, den erfindungsgemäßen medizinischen Kleber an die besonderen Anforderungen verschiedener Einsatzbereiche anzupassen. So wird beispielsweise eine relativ rasche Gelbildung erwünscht sein, wenn im Rahmen einer Operation Blutungen gestillt werden müssen, wohingegen beim Fixieren von Implantaten eine langsamere Gelbildung vorteilhaft sein kann, da sie dem Arzt die Möglichkeit bietet, die genaue Lage des Implantats auch nach dem Verabreichen des Klebers in Ruhe anpassen zu können.

### Beispiel 3: Vernetzung von Gelatine mit Transglutaminase: Einfluss der Menge an Vernetzungsmittel

In diesem Beispiel wurde die thermisch vorbehandelte Gelatine C aus dem Beispiel 1 mit unterschiedlichen Mengen an Transglutaminase vernetzt.

Eine 8 Gew.%ige Lösung der Gelatine C wurde hergestellt wie in Beispiel 1 beschrieben. Für jeden Ansatz wurden 5,9 ml dieser Lösung auf 37°C vorgewärmt und mit der in der nachfolgenden Tabelle 4 angegebenen Menge an Transglutaminase-Stammlösung (30 U/ml, auf 37°C vorgewärmt) versetzt, um die Vernetzungsreaktion zu starten. Die Bestimmung des Gelpunktes anhand der Gelfestigkeitsmessung erfolgte wie in Beispiel 1 beschrieben.

**Tabelle 4**

| Ansatz | 3-1 | 3-2 | 3-3 |
|---|---|---|---|
| Menge an Transglutaminase-Stammlösung | 0,2 ml | 0,3 ml | 0,5 ml |
| Transglutaminase pro Gramm Gelatine | 12,7 Units/g | 19,1 Units/g | 31,8 Units/g |
| Gelpunkt | 6,0 min | 4,0 min | 3,0 min |

Wie aus den in der Tabelle 4 angegebenen Werten hervorgeht, kann die Geschwindigkeit der Bildung des vernetzten Gelatinegels auch über die Konzentration des Vernetzungsmittels, in diesem Fall der Transglutaminase, beeinflusst werden. Erwartungsgemäß führt eine höhere Menge an Vernetzungsmittel zu einer schnelleren Gelbildung.

### Beispiel 4: Vernetzung von Gelatine mit Transglutaminase: Verwendung von Gelatine aus unterschiedlichen Rohstoffquellen

In diesem Beispiel wurde zum einen Gelatine aus Rinderknochen ("bovine limed bone") und zum anderen Fischgelatine, welche unter den Bedingungen des Standard-Bloom-Tests nicht geliert, bei unterschliedlichen Gelatinekonzentrationen mit Transglutaminase vernetzt. Das Ansetzten der Reaktionsmischungen und die Messung der Gelfestigkeit erfolgten wie in Beispiel 1 beschrieben.

Die Gelatinetypen, die Konzentration der eingesetzten Gelatinelösungen, die Zusammensetzung der Reaktionsmischungen und die sich aus der Gelfestigkeitsmessung ergebenden Gelpunkte sind in der folgenden Tabelle 5 dargestellt. Ferner sind die auf die Fläche bezogene Gelfestigkeit und Haftung 10 min nach dem Gelpunkt angegeben.

**Tabelle 5**

| Ansatz | 4-1 | 4-2 | 4-3 | 4-4 |
|---|---|---|---|---|
| Gelatinetyp | Rinderknochen | Rinderknochen | Fischgelatine | Fischgelatine |
| Bloom-Wert | 240 g | 240 g | (nicht gelierend) | (nicht gelierend) |
| Viskosität (6,7 Gew.%, 60°C) | 9,2 mPa·s | 9,2 mPa·s | 2,1 mPa·s | 2,1 mPa·s |
| Konzentration der Gelatinelösung | 8 Gew.% | 8 Gew.% | 15 Gew.% | 15 Gew.% |
| Gelatinelösung | 5,9 ml | 5,4 ml | 5,4 ml | 4,8 ml |
| Transglutaminase Stammlösung | 0,6 ml | 1,0 ml | 1,0 ml | 1,6 ml |
| Transglutaminase pro g Gelatine | 38 Units/g | 69 Units/g | 37 Units/g | 67 Units/g |
| Gelpunkt | 3,0 min | 1,5 min | 14,5 min | 9,3 min |
| Gelfestigkeit nach 10 min | 395 mN/cm² | 870 mN/cm² | 395 mN/cm² | 710 mN/cm² |
| Haftung nach 10 min | 80 mN/cm² | 95 mN/cm² | 195 mN/cm² | 240 mN/cm² |

Die Ergebnisse zeigen, dass durch die Verwendung der niedrigviskosen Fischgelatine im Vergleich zur Rinderknochengelatine deutlich spätere Gelpunkte erzielt werden können, und dies bei einer fast doppelt so hohen Gelatinekonzentration. Trotz der späteren Gelbildung im Fall der Fischgelatine werden dabei jedoch vergleichbare Gelfestigkeiten erreicht, und die Haftung (Klebrigkeit) ist sogar deutlich höher (jeweils 10 min nach dem Gelpunkt). Wie zu erwarten, führt die Erhöhung der Vernetzungsmittelkonzentration bei beiden Gelatinen zu einem früheren Gelpunkt und zu einer höheren Gelfestigkeit (Ansätze 4-2 und 4-4 gegenüber den Ansätzen 4-1 bzw. 4-3).

Ein besonderer Vorteil bei der Verwendung von nicht gelierender Fischgelatine besteht darin, dass die Gelatinelösung bei Raumtemperatur flüssig bleibt und dadurch die Bereitsstellung und Handhabung des medizinischen Klebers vereinfacht werden.

### Durch den Einsatz von Mischungen verschiedener Gelatinetypen, z.B. von

Fischgelatine mit Rinderknochen- oder Schweineschwartengelatine, ergibt sich eine weitere Möglichkeiten (neben der Variation der Gelatine- und der Vernetzungsmittelkonzentration), um den Gelpunkt, die Gelfestigkeit und die Haftung des medizinischen Klebers zu beeinflussen.

### Beispiel 5: Vernetzung von Gelatine mit Transglutaminase: Verwendung eines Gelatine/Carboxymethylcellulose-Blends

Auch in diesem Beispiel wurde eine Vernetzung von Gelatine mit Transglutaminase durchgeführt, wobei zur deutlichen Erhöhung der Ausgangsviskosität der unvernetzten Gelatinelösung zunächst ein Gelatine-Blend hergestellt wurde. Dieser Blend enthielt 92 Gew.% Schweineschwartengelatine (261 g Bloom) und 8 Gew.% Carboxymethylcellulose (CMC, durchschnittlicher Substitutionsgrad 0,7; Viskosität ca. 8.000 mPa·s in 1 Gew.%iger Lösung bei 25°C). Der Gelatine/CMC-Blend wies einen Bloom-Wert von 260 g und eine Viskosität von 162 mPa·s (6,7 Gew.% bei 60 °C) auf.

Das Ansetzen der Reaktionsmischung und die Messung der Gelfestigkeit erfolgten wie in Beispiel 1 beschrieben, wobei zunächst 5,0 ml einer 8 Ges.%-igen Lösung des Blends hergestellt und dann mit 0,3 ml Transglutaminase-Stammlösung und 0,9 ml destilliertem Wasser versetzt wurden (dies entspricht 22,5 Units Transglutaminase pro Gramm Gelatine/CMC-Blend).

Der Gelpunkt lag in diesem Versuch bei 4,0 min. 10 min nach dem Gelpunkt wurden eine Gelfestigkeit von 350 mN/cm² und eine Haftung von 70 mN/cm² gemessen.

Ein Vergleich mit den Ansätzen 2-2 und 3-2 zeigt, dass trotz der deutlichen Erhöhung der Ausgangsviskosität der Gelatinelösung durch den Zusatz von CMC die Kinetik der Vernetzungsreaktion hierdurch nicht wesentlich beeinflusst wird. Dies eröffnet eine zusätzliche Möglichkeit, den medizinischen Kleber an die jeweiligen Erfordernisse anzupassen: Mit Hilfe eines Gelatine/CMC-Blends kann eine Lösung bereitgestellt werden, die bereits vor der Vernetzung sehr viskos, aber dennoch fließfähig ist, was z.B. beim Verschließen von Nervenleitschienen von großem Vorteil ist.

### Beispiel 6: Vernetzung von Gelatine mit Transglutaminase: Verwendung einer partiell vernetzten Gelatine

In diesem Beispiel wurde die Gelatine zunächst einem partiellen (ersten) Vernetzungsschritt unterzogen, um die Ausgangsviskosität der Gelatinelösung zu erhöhen und um eine deutlich schnellere Gelbildung im eigentlichen (in diesem Fall zweiten) Vernetzungsschritt zu erzielen.

Als Ausgangsmaterials für die Herstellung der partiell vernetzten Gelatine diente eine Gelatine aus Schweineknochen mit einem Bloomwert von 250 g und einer Viskosität von 6,6 mPa•s (6,7 Gew.-% bei 60 °C). Eine 10 Gew.-%ige Lösung dieser Gelatine in destilliertem Wasser wurde angesetzt, indem die Gelatine zunächst 45 Minuten bei Raumtemperatur gequollen und dann für eine Stunde bei 60 °C gelöst wurde. Anschließend wurde die Lösung auf 50 °C temperiert und die entsprechende Menge der Transglutaminase-Stammlösung (30 Units/ml) zugegeben, sodass eine Menge von 1,5 Units Transglutaminase pro Gramm Gelatine vorlagen. Zur Durchführung der partiellen Vernetzung wurde die Lösung für 2 Stunden unter Rühren bei 50 °C gehalten.

Zum Abstoppen der Vernetzungsreaktion wurde die Transglutaminase durch Erwärmen der Lösung auf 80 °C thermisch deaktiviert, anschließend wurde die Lösung sofort im Eisbad abgekühlt, in eine Schale gegossen und gelieren gelassen. Das erhaltene Gelatinegel wurde gewolft, bei 20 °C und einer relativen Luftfeuchtigkeit von 10 % getrocknet und anschließend gemahlen. Die auf diese Weise erhaltene partiell vernetzte Gelatine wird im Folgenden als P2 bezeichnet.

Eine weitere partiell vernetzte Gelatine mit einem etwas höheren Vernetzungsgrad wurde wie vorstehend beschrieben hergestellt, mit der Abweichung, dass die partielle Vernetzungsreaktion für 3 Stunden durchgeführt wurde. Diese Gelatine wird im Folgenden als P3 bezeichnet.

Die Bloomwerte und Viskositäten bei 60 °C und 37 °C der Ausgangsgelatine P0 und der partiell vernetzten Gelatinen P2 und P3 sind in der nachfolgenden Tabelle 6 dargestellt.

**Tabelle 6**

| Gelatine | P0 (Vergleichsbsp.) | P2 | P3 |
|---|---|---|---|
| Bloom-Wert | 250 g | 228 g | 257 g |
| Viskosität | 6,6 mPa•s | 9,6 mPa•s | 16,7 mPa•s |
| (6,7 Gew.-%, 60 °C) | | | |
| Viskosität | 28,6 mPa•s | 69,8 mPa•s | 200 mPa•s |
| (10 Gew.-%, 37 °C) | | | |

Durch die partielle Vernetzung konnte die Viskosität der Gelatine bei 60 °C um das ca. 1,5-fache (P2) bzw. das ca. 2,5-fache (P3) gegenüber der unvernetzten Gelatine (P0) erhöht werden. Noch wesentlich deutlicher wirkt sich die Viskositätserhöhung bei 37 °C, d.h. bei einer bevorzugten Applikationstemperatur des medizinischen Klebers, aus. Hier erhöhte sich die Viskosität um das ca. 2,5-fache bzw. das ca. 7-fache.

Unter Verwendung der Gelatinen P0, P2 bzw. P3 wurde eine Vernetzungsreaktion mit Transglutaminase durchgeführt und die Gelfestigkeit und Klebrigkeit in Abhängigkeit von der Reaktionszeit bestimmt, wie dies in Beispiel 1 beschrieben wurde. Es wurde jeweils von 5 ml einer 10 Gew-%igen Gelatinelösung ausgegangen, der jeweils 1,2 ml der Transglutaminase-Stammlösung (30 Units/ml) zugegeben wurden. Dies entspricht einer Enzymmenge von 72 Units pro Gramm Gelatine.

Die Messergebnisse sind in der nachstehenden Tabelle 7 aufgeführt.

**Tabelle 7**

| Ansatz | P0 (Vergleichsbsp.) | P2 | P3 |
|---|---|---|---|
| Gelpunkt | 1,5 min | 50 s | < 5 s |
| Gelfestigkeit nach 10 min | 1184 mN/cm² | 592 mN/cm² | 631 mN/cm² |
| Haftung nach 10 min | 316 mN/cm² | 197 mN/cm² | 237 mN/cm² |

Es zeigt sich, dass durch die partielle Vernetzung der Gelatine der Gelpunkt des vernetzten Gelatinegels wesentlich schneller erreicht werden kann. Bemerkenswert ist insbesondere das mit der Gelatine P3 erzielte Ergebnis, d.h. eine fast sofortige Gelbildung innerhalb von weniger als 5 s nach dem Mischen der Gelatine mit der Transglutaminase.

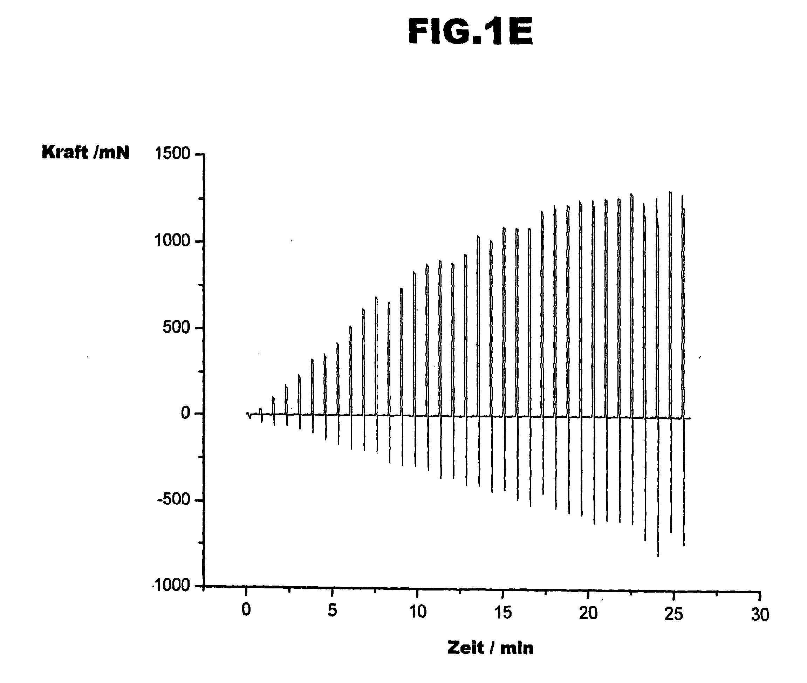

In der Figur 1E ist die gemessene Kraft in Abhängigkeit von der Reaktionszeit für den Ansatz mit der Gelatine P3 dargestellt (Durchführung der Messung wie in Beispiel 1 beschrieben). Aus der Abbildung wird deutlich, dass trotz der fast unmittelbar nach dem Mischen erfolgenden Gelierung die Gelfestigkeit und die Haftung kontinuierlich ansteigen und erst einige Zeit nach dem Gelpunkt ihren maximalen Wert erreichen. Dieser Effekt ist für die Anwendung der vorliegenden Erfindung äußerst vorteilhaft und ermöglicht es, dass der medizinische Kleber während einem gewissen Zeitraum nach der Applikation noch plastisch verformbar ist und an die Struktur des Applikationsbereichs angepasst werden kann.

### Beispiel 7: Nachweis der Haftkraft des medizinischen Klebers beim Verkleben von Gewebe in vivo

In diesem Beispiel wurde unter klinischen Bedingungen die Klebewirkung des erfindungsgemäßen medizinischen Klebers an vitalem Gewebe getestet. Dabei wurden die in Beispiel 1 beschriebene Gelatine C und Transglutaminase als Vernetzungsmittel verwendet.

Es wurde eine 12 Gew.-%ige Gelatinelösung in PBS-Puffer wie in Beispiel 1 beschrieben hergestellt und in die erste Kammer einer Doppelkammerspritze gefüllt. In die zweite Kammer der Spritze wurde eine entsprechende Menge der in Beispiel 1 beschriebenen Transglutaminase-Stammlösung gefüllt, so dass eine Menge von 30 Units Transglutaminase pro Gramm Gelatine vorlag.

Für den Praxistest wurde einer narkotisierten Maus (Blab/c Maus) die ventrale Subcutis von der Faszie abgehoben und anschließend mehrere Gewebeabschnitte der Haut mit einer Fläche von jeweils ca. 1 cm² präpariert. Danach wurden die beiden Komponenten des medizinischen Klebers, die zuvor in der Doppelkammerspritze auf 37°C erwärmt worden waren, miteinander vermischt und gleichzeitig in einer Menge von ca. 0,2 ml/cm² auf die Faszie aufgetragen. Die präparierte Subcutis wurde passgenau eingefügt und durch leichtes Andrücken fixiert. Anschließend wurde die Verklebung mechanisch auf Zugbelastung geprüft. Nach einer Klebdauer von ca. 4 Minuten nach dem Auftragen des Klebers konnte an den einzelnen Gewebeabschnitten ein stabiler Verbund zwischen Subcutis und Faszie festgestellt werden.

Der Versuch wurde mehrmals mit dem gleichen Ergebnis wiederholt, wobei keinerlei sonstige Komplikationen auftraten.

### Beispiel 8: Einsatz des medizinischen Klebers in vivo als Wundverschluss

Um zu prüfen, ob sich der erfindungsgemäße medizinische Kleber unter klinischen Bedingungen zur Blutstillung diffuser Blutungen eignet, wurde an einer narkotisierten Maus (Blab/c Maus) eine atypische Keilresektion am linken Leberlappen ausgeführt. Auf die blutende Schnittfläche mit einer Länge von ca. 0,6 cm wurden ca. 0,1 ml des in Beispiel 6 beschriebenen medizinische Klebers unter Verwendung einer Doppelkammerspritze aufgetragen. Innerhalb von 5 Minuten nach Einsatz des Klebers wurde eine vollständige Blutungsstillung erreicht.

Der Versuch wurde mehrmals mit dem gleichen Ergebnis wiederholt, wobei keinerlei sonstige Komplikationen auftraten.

### Beispiel 9: Herstellung und Auflösungsverhalten einer lyophilisierten Feststoffmischung aus Gelatine und Transglutaminase

Dieses Beispiel beschreibt die Herstellung einer Feststoffmischung, die 6 Units Transglutaminase pro Gramm Gelatine enthält.

75 g der Gelatine A aus dem Beispiel 1 (Schweineschwartengelatine mit 290 g Bloom) wurden in 425 g destilliertem Wasser gequollen und bei 60°C gelöst. Die Lösung wurde auf 45°C abkühlen gelassen, mit 15 ml der Transglutaminase-Stammlösung (30 U/ml, siehe Beispiel 1) versetzt und gut durchmischt. Zwei Gefriertrocknungsschalen wurden mit flüssigem Stickstoff gekühlt, die Gelatine und Transglutaminase enthaltende Lösung darin verteilt und mittels flüssigem Stickstoff eingefroren. Die eingefrorene Lösung wurde zwei Tage in einer Gefriertrocknungsanlage vom Typ Lyovac GT 2-s (Hersteller: AMSCO Finn-Aqua GmbH, Hürth) lyophilisiert.

Die erhaltene lyophilisierte Feststoffmischung wurde unter ständiger Kühlung mit flüssigem Stickstoff in einem Mörser zu einem feinen Pulver vermahlen und anschließend unter Vakuum getrocknet. Da das Pulver stark hygroskopisch ist, wurde es unter Luftabschluss bei ca. 4°C gelagert.

Eine weitere Feststoffmischung wurde hergestellt, indem die beschriebene Vorgehensweise mit denselben Mengenverhältnissen, aber unter Verwendung einer kaltwasserlöslichen Instantgelatine an Stelle der Gelatine A wiederholt wurde. Diese Instantgelatine enthält zur Verbesserung ihrer Löslichkeit ca. 15 Gew.% an niedermolekularem Gelatinehydrolysat.

Das Auflösungsverhalten der auf diese Weise hergestellten Feststoffmischungen wurde wie folgt untersucht: jeweils 50 mg Feststoffmischung wurden in ein verschließbares Röhrchen eingewogen und mit 950 µl PBS-Puffer (pH 7,2), der auf 37°C vorgewärmt war, versetzt. Die Röhrchen wurden mit Hilfe eines Reagenzglasschüttlers geschüttelt und die Zeit bis zur sichtbaren Auflösung der Feststoffmischung bestimmt.

Die aus der Gelatine A hergestellte Mischung war nach 2,7 min, die aus der Instantgelatine hergestellte Mischung bereits nach 2 min aufgelöst.

Das Beispiel zeigt, dass lyophilisierte Gelatine bei 37°C oder niedriger in einer wässrigen Lösung aufgelöst werden kann. Dies ist darauf zurückzuführen, dass die Gelatine als Ergebnis des Gefriertrocknungsprozesses überwiegend in amorpher Form vorliegt. Durch die Verwendung von Instantgelatine kann die Auflösegeschwindigkeit weiter verbessert werden.

Derartige lyophilisierte Feststoffmischungen aus Gelatine und einem Vernetzungsmittel können im Rahmen der vorliegenden Erfindung vorteilhaft eingesetzt werden. Die Mischung kann bei Raumtemperatur oder gekühlt bereitgestellt werden und dann vom behandelnden Arzt in einer wässrigen Lösung bei 37°C oder niedriger aufgelöst werden.

### Vergleichsbeispiel:

### Vergleich der Gelbildung bei einer auf Thrombin und Fibrinogen basierenden Zusammensetzung

Zum Vergleich mit der vorliegenden Erfindung wurde die Kinetik der Gelbildung bei einem handelsüblichen medizinischen Kleber auf der Basis von Thrombin und Fibrinogen untersucht. Dieser Fibrinkleber beruht auf dem Prinzip der natürlichen Blutgerinnung und umfasst zwei zu mischende Komponenten mit folgender Zusammensetzung:
1. Komponente: Kleberproteinlösung, enthaltend:
   - Humanplasmaproteinfraktion mit Fibrinogen
   - Blutgerinnungsfaktor XIII
   - Plasmafibronectin
   - Aprotinin (bovin)
2. Komponente: Thrombinlösung, enthaltend:
   - Thrombin (human)
   - Calciumchlorid

Zur Bestimmung der Gelbildungskinetik des Fibrinklebers wurden beide Komponenten für 20 min auf 37°C vorgewärmt und dann in einem zylindrischen Gefäß (Durchmesser 3 cm) gemischt, das mit Hilfe eines Aluminiumblockes auf 37°C temperiert wurde. Die Mischung der Komponenten definiert den Startzeitpunkt (0 min). Die Messung der Gelfestigkeit und Klebrigkeit (Haftung) der Zusammensetzung in Abhängigkeit von der Reaktionszeit mittels der Kraft/ Weg-Messvorrichtung vom Typ Zwick BZ 2.5/TN1S erfolgte wie oben beschrieben.

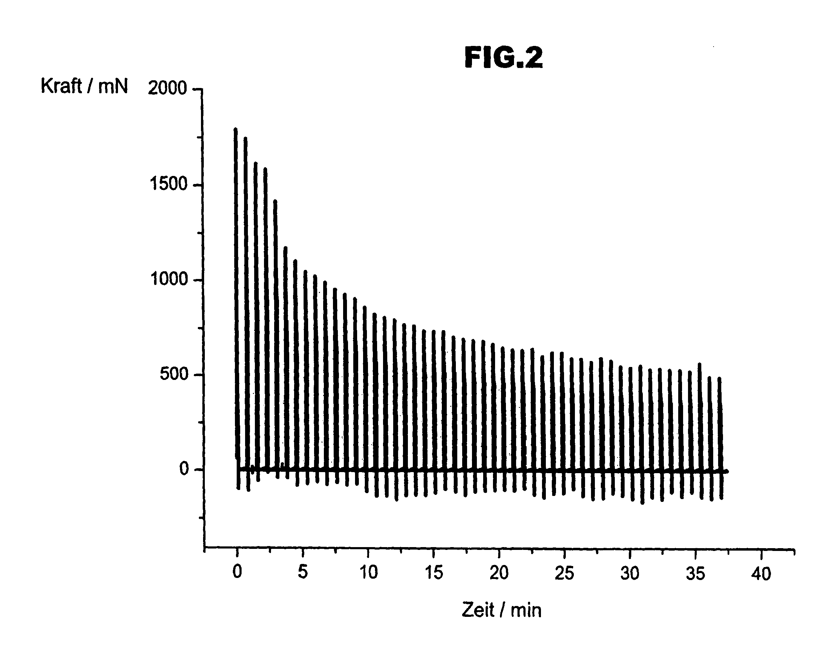

Das Ergebnis der Messung ist in dem Diagramm in der Figur 2 dargestellt. Beim Vergleich mit den Figuren 1A bis 1D wird sofort deutlich, dass die Gelbildung dem Fibrinkleber einen gänzlich anderen Verlauf aufweist als bei dem erfindungsgemäßen medizinischen Kleber: Die Zusammensetzung wird praktisch schlagartig fest, d.h. die Gelfestigkeit erreicht innerhalb weniger Sekunden ihren maximalen Wert. Danach fällt die Gelfestigkeit wieder ab (auf weniger als die Hälfte des Anfangswertes innerhalb von 35 min). Das Gel weist eine deutliche Synerese auf.

Ein weiterer Vorteil des erfindungsgemäßen Klebers beruht auf der Verfügbarkeit der beiden Komponenten Gelatine und Vernetzungsmittel in hoher und reproduzierbarer Qualität. Dadurch ist auch die Kinetik der Gelbildung bei gegebener quantitativer Zusammensetzung in hohem Maße reproduzierbar. Im Gegensatz hierzu wurden bei dem untersuchten Fibrinkleber, in Abhängigkeit von der jeweiligen Charge, zum Teil deutliche Abweichungen bei der Gelbildung festgestellt, was unter anderem auf dem humanen Ursprung einiger Komponenten, die in ihrer Qualität einer natürlichen Schwankung unterliegen, beruhen dürfte.

Auch bei der Klebrigkeit zeigen sich deutliche Unterschiede. Die Haftung des Fibrinklebers an der Kunststoffoberfläche des eingesetzten Stempels ist während des Messzeitraums von 35 min in Figur 2 im Wesentlichen konstant, während bei den erfindungsgemäßen Klebern aus Beispiel 1 ein Anstieg der Haftung, der mit dem Anstieg der Gelfestigkeit einhergeht, zu beobachten ist.

Mit Ausnahme des Ansatzes A wird aber auch eine merklich höhere Haftung erzielt als bei dem Fibrinkleber, was einen weiteren Vorteil der vorliegenden Erfindung deutlich macht. Selbst gegenüber einer glatten Kunststoffoberfläche, wie sie in diesem Versuch eingesetzt wurde, kann eine sehr gute Haftung erzielt werden, beispielsweise über 200 mN/cm² beim Ansatz C. Der erfindungsgemäße medizinische Kleber eignet sich daher nicht nur zum Kleben von Gewebe, sondern z.B. auch von Implantaten, die eine glatte Oberfläche aufweisen.

## Patentansprüche

1. Verwendung von Gelatine und Transglutaminase zur Herstellung eines vernetzenden medizinischen Klebers, der ein vernetztes Gelatinegel in einem Applikationsbereich des menschlichen oder tierischen Körpers bildet, wobei
(i) die Gelatine und die Transglutaminase zur Bildung des vernetzenden medizinischen Klebers miteinander gemischt werden, welcher anschließend in den Applikationsbereich verabreicht wird; oder
(ii) die Gelatine und die Transglutaminase in voneinander getrennter Form bereitgestellt und gleichzeitig oder aufeinander folgend unter Bildung des vernetzenden medizinischen Klebers in den Applikationsbereich verabreicht werden,
**dadurch gekennzeichnet, dass** die Gelatine eine Viskosität von 7 mPa•s oder mehr aufweist, gemessen in einer 6,7 Gew.%igen Lösung bei 60 °C.

2. Verwendung nach Anspruch 1, wobei die Gelatine eine Fischgelatine ist.

3. Verwendung nach einem der vorangehenden Ansprüche, wobei der medizinische Kleber gemäß (i) eine wässrige Lösung ist, die die Transglutaminase und die Gelatine gelöst enthält.

4. Verwendung nach Anspruch 3, wobei die wässrige Lösung durch Auflösen einer Feststoffmischung, welche die Gelatine und die Transglutaminase in lyophilisierter Form umfasst, hergestellt wird.

5. Verwendung nach Anspruch 4, wobei die Gelatine zumindest überwiegend in amorpher Form vorliegt.

6. Verwendung nach Anspruch 4 oder 5, wobei die Transglutaminase in einer Menge von 0,6 bis 80 Units pro Gramm Gelatine in der Feststoffmischung enthalten ist.

7. Verwendung nach Anspruch 6, wobei die Transglutaminase in einer Menge von 10 bis 40 Units pro Gramm Gelatine in der Feststoffmischung enthalten ist.

8. Verwendung nach Anspruch 1 oder 2, wobei das Bereitstellen gemäß (ii) in Form einer wässrigen Gelatinelösung und einer separaten wässrigen Transglutaminase-Lösung erfolgt.

9. Verwendung nach Anspruch 8, wobei die gleichzeitige Verabreichung der Gelatinelösung und der Transglutaminase-Lösung durch Injektion mit einer Mehrkammerapplikationsvorrichtung erfolgt.

10. Verwendung nach Anspruch 1 oder 2, wobei das Bereitstellen gemäß (ii) in Form einer wässrigen Gelatinelösung und der Transglutaminase in fester Form erfolgt.

11. Verwendung nach einem der vorangehenden Ansprüche, wobei die Gelatinekonzentration in dem medizinischen Kleber 5 bis 20 Gew.% beträgt.

12. Verwendung nach einem der vorangehenden Ansprüche, wobei das vernetzte Gelatinegel eine Gelfestigkeit von 100 g oder mehr aufweist, gemessen mit einem Stempel mit einem Durchmesser von 12,7 mm bei einer Eindringtiefe von 4 mm.

13. Verwendung nach einem der vorangehenden Ansprüche, wobei die Haftung des vernetzten Gelatinegels an einer glatten Kunststoffoberfläche 200 mN/cm² oder mehr beträgt.

14. Verwendung nach einem der vorangehenden Ansprüche, wobei der medizinische Kleber ein viskositätserhöhendes Polymer mit einem Anteil von 1 bis 10 Gew.%, bezogen auf die Feststoffanteile des Klebers, umfasst.

15. Verwendung nach Anspruch 14, wobei das viskositätserhöhende Polymer Carboxymethylzellulose umfasst.

16. Verwendung nach einem der vorangehenden Ansprüche, wobei die Gelatine vor dem Mischen und/oder Verabreichen gemäß (i) oder (ii) partiell vernetzt ist.

17. Verwendung nach einem der vorangehenden Ansprüche, wobei der medizinische Kleber einen oder mehrere therapeutische Wirkstoffe umfasst.

18. Verwendung nach einem der vorangehenden Ansprüche zur Herstellung eines vernetzenden medizinischen Klebers zum Stillen von Blutungen, wobei der medizinische Kleber auf einen von der Blutung betroffenen Applikationsbereich aufgebracht wird, wobei durch die Bildung des vernetzten Gelatinegels die Blutung gestillt wird.

19. Verwendung nach einem der Ansprüche 1 bis 17 zur Herstellung eines vernetzenden medizinischen Klebers zur Behandlung von Verletzungen und/oder Verbrennungen der menschlichen oder tierischen Haut.

20. Verwendung nach einem der Ansprüche 1 bis 17 zur Herstellung eines vernetzenden medizinischen Klebers zur Fixierung autologer oder allogener Gewebe in einem Applikationsbereich des menschlichen oder tierischen Körpers.

21. Verwendung nach einem der Ansprüche 1 bis 17 zur Herstellung eines vernetzenden medizinischen Klebers zur Fixierung von Implantaten in einem Applikationsbereich des menschlichen oder tierischen Körpers.

22. Feststoffmischung, welche Gelatine und Transglutaminase in lyophilisierter Form umfasst, **dadurch gekennzeichnet, dass** die Gelatine eine Viskosität von 7 mPa • s oder mehr aufweist, gemessen in einer 6,7 Gew.%igen Lösung bei 60 °C.

23. Mehrkammerapplikationsvorrichtung, welche eine wässrige Gelatinelösung und eine wässrige Transglutaminase-Lösung in getrennten Kammern enthält, **dadurch gekennzeichnet, dass** die Gelatine eine Viskosität von 7 mPa • s oder mehr aufweist, gemessen in einer 6,7 Gew.%igen Lösung bei 60 °C.

## Claims

1. Use of gelatin and transglutaminase for producing a cross-linking medical glue, which forms a cross-linked gelatin gel in an area of application of the human or animal body, wherein
i. the gelatin and the transglutaminase are mixed with one another to form the cross-linking medical glue, which is then administered to the area of application; or
ii. the gelatin and the transglutaminase are provided in separate form and are administered simultaneously or consecutively to the area of application with the formation of the cross-linking medical glue,
**characterized in that** the gelatin has a viscosity of 7 mPa·s or more, measured in a 6.7% by wt. aqueous solution at 60°C.

2. Use according to claim 1, wherein the gelatin is a fish gelatin.

3. Use according to one of the preceding claims, wherein the medical glue according to (i) is an aqueous solution, which contains the transglutaminase and the gelatin in solution.

4. Use according to claim 3, wherein the aqueous solution is produced by dissolving a solid mixture, which comprises the gelatin and the transglutaminase in lyophilised form.

5. Use according to claim 4, wherein the gelatin is present at least predominantly in amorphous form.

6. Use according to claim 4 or 5, wherein the transglutaminase is contained in the solid mixture in a quantity of 0.6 to 80 units per gram of gelatin.

7. Use according to claim 6, wherein the transglutaminase is contained in the solid mixture in a quantity of 10 to 40 units per gram of gelatin.

8. Use according to claim 1 or 2, wherein the variant according to (ii) is provided in the form of an aqueous gelatin solution and a separate aqueous transglutaminase solution.

9. Use according to claim 8, wherein the simultaneous administration of the gelatin solution and the transglutaminase solution is conducted by injection with a multi-chamber applicator.

10. Use according to claim 1 or 2, wherein the variant according to (ii) is provided in the form of an aqueous gelatin solution and the transglutaminase in solid form.

11. Use according to one of the preceding claims, wherein the gelatin concentration in the medical glue amounts to 5 to 20% by wt.

12. Use according to one of the preceding claims, wherein the cross-linked gelatin gel has a gel strength of 100 g or more, measured with a plunger with a diameter of 12.7 mm at a penetration depth of 4 mm.

13. Use according to one of the preceding claims, wherein the adhesion of the cross-linked gelatin gel on a smooth plastic surface amounts to 200 mN/cm² or more.

14. Use according to one of the preceding claims, wherein the medical glue comprises a viscosity-increasing polymer with a proportion of 1 to 10% by wt., based on the solid contents of the glue.

15. Use according to claim 14, wherein the viscosity-increasing polymer is carboxymethylcellulose.

16. Use according to one of the preceding claims, wherein the gelatin is partially cross-linked.

17. Use according to one of the preceding claims, wherein the medical glue comprises one or more therapeutic active substances.

18. Use according to one of the preceding claims for producing a cross-linking medical glue for stopping haemorrhaging, wherein the medical glue is applied to an area of application affected by the haemorrhaging, wherein the haemorrhaging is stopped by the formation of the cross-linked gelatin gel.

19. Use according to one of claims 1 to 17 for producing a cross-linking medical glue for the treatment of injuries and/or burns of human or animal skin.

20. Use according to one of claims 1 to 17 for producing a cross-linking medical glue for fixing autologous or allogenic tissue in an area of application of the human or animal body.

21. Use according to one of claims 1 to 17 for producing a cross-linking medical glue for fixing implants in an area of application of the human or animal body.

22. Solid mixture comprising gelatin and transglutaminase in lyophilised form, **characterized in that** the gelatin has a viscosity of 7 mPa·s or more, measured in a 6.7% by wt. aqueous solution at 60°C.

23. Multi-chamber applicator, which contains an aqueous gelatin solution and an aqueous transglutaminase solution in separate chambers, **characterized in that** the gelatin has a viscosity of 7 mPa·s or more, measured in a 6.7% by wt. aqueous solution at 60°C.

## Revendications

1. Utilisation de gélatine et de transglutaminase pour la préparation d'un adhésif médical à réticulation, lequel forme un gel de gélatine réticulé dans un domaine d'application du corps humain ou animal, dans laquelle
(i) la gélatine et la transglutaminase sont mélangées ensemble pour la formation de l'adhésif médical à réticulation, lequel est ensuite administré dans le domaine de l'application ; ou
(ii) la gélatine et la transglutaminase sont préparées dans une forme séparée l'une de l'autre et sont, simultanément ou l'une après l'autre, administrées dans le domaine de l'application avec la formation de l'adhésif médical à réticulation,
**caractérisée en ce que** la gélatine présente une viscosité de 7 mPa.s ou supérieure, mesurée dans une solution à 6,7 % en masse à 60°C.

2. Utilisation selon la revendication 1, dans laquelle la gélatine est une gélatine de poisson.

3. Utilisation selon l'une quelconque des revendications précédentes, dans laquelle l'adhésif médical selon (i) est une solution aqueuse qui contient la transglutaminase et la gélatine dissoutes.

4. Utilisation selon la revendication 3, dans laquelle la solution aqueuse est préparée par dissolution d'un mélange de matières solides, lequel comprend la gélatine et la transglutaminase dans une forme lyophilisée.

5. Utilisation selon la revendication 4, dans laquelle la gélatine est présente au moins de manière prédominante dans une forme amorphe.

6. Utilisation selon la revendication 4 ou 5, dans laquelle la transglutaminase est contenue dans une quantité de 0,6 à 80 unités par gramme de gélatine dans le mélange de matières solides.

7. Utilisation selon la revendication 6, dans laquelle la transglutaminase est contenue dans une quantité de 10 à 40 unités par gramme de gélatine dans le mélange de matières solides.

8. Utilisation selon la revendication 1 ou 2, dans laquelle la préparation selon (ii) est réalisée dans la forme d'une solution aqueuse de gélatine et d'une solution aqueuse séparée de transglutaminase.

9. Utilisation selon la revendication 8, dans laquelle l'administration simultanée de la solution de gélatine et de la solution de transglutaminase est réalisée par infection avec un dispositif d'application à chambres multiples.

10. Utilisation selon la revendication 1 ou 2, dans laquelle la préparation selon (ii) est réalisée dans la forme d'une solution aqueuse de gélatine et de la transglutaminase dans une forme solide.

11. Utilisation selon l'une quelconque des revendications précédentes, dans laquelle la concentration en gélatine dans l'adhésif médical est de 5 à 20 % en masse.

12. Utilisation selon l'une quelconque des revendications précédentes, dans laquelle le gel de gélatine réticulé présente une solidité de gel de 100 g ou supérieure, mesurée avec un tampon d'un diamètre de 12,7 mm pour une profondeur de pénétration de 4 min.

13. Utilisation selon l'une quelconque des revendications précédentes, dans laquelle l'adhérence du gel de gélatine réticulé sur une surface lisse de matière plastique est de 200 mN/cm² ou supérieure.

14. Utilisation selon l'une quelconque des revendications précédentes, dans laquelle l'adhésif médical comprend un polymère élevant la viscosité avec une part de 1 à 10 % en masse, rapportée à la part en matière solide de l'adhésif.

15. Utilisation selon la revendication 14, dans laquelle le polymère élevant la viscosité comprend de la carboxyméthylcellulose.

16. Utilisation selon l'une quelconque des revendications précédentes, dans laquelle la gélatine est, avant le mélange et/ou l'administration selon (i) ou (ii), partiellement réticulée.

17. Utilisation selon l'une quelconque des revendications précédentes, dans laquelle l'adhésif médical comprend un ou plusieurs principes actifs thérapeutiques.

18. Utilisation selon l'une quelconque des revendications précédentes pour la préparation d'un adhésif médical à réticulation destiné à l'arrêt de saignements, dans laquelle l'adhésif médical est appliqué sur un domaine d'application concerné par le saignement, sur quoi le saignement est arrêté par la formation du gel de gélatine réticulé.

19. Utilisation selon l'une quelconque des revendications 1 à 17 pour la préparation d'un adhésif médical à réticulation destiné au traitement de blessures et/ou de brûlures de la peau humaine ou animal.

20. Utilisation selon l'une quelconque des revendications 1 à 17 pour la préparation d'un adhésif médical à réticulation destiné à la fixation de tissus autologues ou allogènes dans un domaine d'application du corps humain ou animal.

21. Utilisation selon l'une quelconque des revendications 1 à 17 pour la préparation d'un adhésif médical à réticulation destiné à la fixation d'implants dans un domaine d'application du corps humain ou animal.

22. Mélange de matières solides, lequel comprend de la gélatine et de la transglutaminase dans une forme lyophilisée, **caractérisé en ce que** la gélatine présente une viscosité de 7 mPa.s ou supérieure, mesurée dans une solution à 6,7 % en masse à 60°C.

23. Dispositif d'application à chambres multiples, lequel contient une solution aqueuse de gélatine et une solution aqueuse de transglutaminase dans des chambres séparées, **caractérisé en ce que** la gélatine présente une viscosité de 7 mPa.s ou supérieure, mesurée dans une solution à 6,7 % en masse à 60°C.
